# EUROPÄISCHE PATENTSCHRIFT

(11) **EP 4 208 750 B1**
(45) Veröffentlichungstag und Bekanntmachung des Hinweises auf die Patenterteilung: **02.10.2024**
(21) Anmeldenummer: 21772754.4
(22) Anmeldetag: 31.08.2021
(51) Int. Cl.: G02B 21/00, A61B 90/20, G02B 21/36, G02B 21/24, A61B 17/00

(54) **VERFAHREN ZUM BETREIBEN EINES MIKROSKOPIESYSTEMS UND MIKROSKOPIESYSTEMS**
METHOD FOR OPERATING A MICROSCOPY SYSTEM, AND MICROSCOPY SYSTEM
PROCÉDÉ DE FONCTIONNEMENT D'UN SYSTÈME DE MICROSCOPIE ET SYSTÈME DE MICROSCOPIE

(30) Priorität: 03.09.2020 DE 102020211133
(43) Veröffentlichungstag der Anmeldung: 12.07.2023
(73) Patentinhaber: Carl Zeiss Meditec AG, 07745 Jena (DE)
(72) Erfinder: VOIGT, Christian, 73453 Abtsgmünd (DE); PHILIPP, Markus, 73430 Aalen (DE)
(74) Vertreter: Ramrath, Lukas
(86) Internationale Anmeldenummer: PCT/EP2021/074011
(87) Internationale Veröffentlichungsnummer: WO 2022/049069

(56) Entgegenhaltungen:
- EP-A2- 1 333 306
- WO-A1-2018/088203
- DE-T5- 112017 005 655
- JP-A- 2003 310 638
- US-A1- 2004 236 352

## Beschreibung

Die Erfindung betrifft ein Verfahren zum Betreiben eines Mikroskopiesystems sowie eine Mikroskopiesystems.

Zur vergrößernden Darstellung von Untersuchungsobjekten werden oft Mikroskope verwendet. In medizinischen Anwendungen, insbesondere zur Unterstützung von chirurgischen Eingriffen, werden so genannte Operationsmikroskope eingesetzt. Diese dienen u.a. zur vergrößernden Darstellung von Teilbereichen eines Körpers, um einem Chirurgen bei einem Eingriff eine bessere visuelle Orientierung zu ermöglichen. Diese Operationsmikroskope sind in der Regel beweglich gehaltert, insbesondere an einem Stativ. Dies ermöglicht es einem Benutzer unter anderem, eine Lage, also eine Position und/oder Orientierung, des Mikroskops zu verändern, beispielsweise um einen Blickwinkel auf einen Untersuchungsbereich zu verändern oder um andere Untersuchungsbereiche zu betrachten.

Bei der Verwendung solcher Mikroskopiesysteme kann es erforderlich sein, eine regelmäßige Umpositionierung vorzunehmen. Hierbei ist es notwendig, bis zu sieben Freiheitsgrade einzustellen, um einen gewünschten Blick auf den Situs zu erhalten, nämlich drei translatorischen Freiheitsgrade, drei rotatorische Freiheitsgrade sowie die Einstellung eines variablen Arbeitsabstands.

Für die Anwendung, einen bestimmten Arbeitspunkts aus verschiedenen Blickwinkeln zu betrachten existieren im Stand der Technik mehrere Lösungen. So beschreibt die EP 1 537 830 A1 ein Verfahren und eine Vorrichtung zum Beobachten von Objekten aus verschiedenen Blickrichtungen mit einem Mikroskop. Hierbei wird eine erste Aufnahme des Zielgebiets des Objekts erstellt und gespeichert, das Mikroskop um einen definierten Winkel verschwenkt, eine zweite Aufnahme erstellt und gespeichert und dann in beiden Aufnahmen ein und derselbe Teil des Objekts markiert. Weiter werden durch Triangulation die Koordinaten des Teils des Objekts berechnet und die Steuerung des Mikroskops so eingerichtet, dass eine Schwenkbewegung um diesen Punkt durchführbar ist.

Ein weiteres System ist aus der JP 2003 310638 A bekannt.

Weiter bekannt ist die US 2006/0274444A1, die ein Mikroskopiesystem offenbart, welches eine Mikroskopoptik mit mindestens einer Linse und einer optischen Achse dieser Linse umfasst, wobei sich die optische Achse mit einer Objektebene in einer Fokusregion schneidet, wobei eine Steuereinrichtung versucht, eine Position des Schnittpunkts beim Betrieb des Mikroskopiesystems konstant zu halten. Weiter offenbart diese Druckschrift, dass auch ein Punkt außerhalb der Fokusregion beim Betrieb des Mikroskopiesystems konstant gehalten werden kann. Da die Objektebene gemäß der Lehre dieser Druckschrift in der Fokusregion liegt, ist die Lage der Objektebene fokuslagenabhängig.

Weiter bekannt ist eine sogenannte Point-Lock-Funktion. Hierbei wird das Mikroskopiesystems um einen Fokuspunkt bewegt. Hierbei existieren verschiedene Varianten. In einer ersten Variante wird das Mikroskopiesystems bzw. das Mikroskop des Mikroskopiesystems bspw. durch einen Joystick positionsgesteuert entlang einer Kugeloberfläche bewegt, wobei gleichzeitig die Orientierung des Mikroskops derart eingestellt wird, dass zu jedem Zeitpunkt die optische Achse den Fokuspunkt schneidet. In einer zweiten Variante bewegt ein Nutzer das Mikroskop, wobei die Orientierung sowie der Fokus automatisch nachgeführt werden, sodass die optische Achse den Fokuspunkt schneidet.

Bei aktivierter Point-Lock-Funktion kann der Nutzer den Fokus zwar manuell verändern, jedoch kann der Drehpunkt nicht verändert werden und bleibt insbesondere ortsfest dort, wo er bei der Aktivierung durch die Fokuslage festgelegt wurde.

In bestimmten Anwendungsfällen ist es wünschenswert, eine Drehbewegung des Mikroskopiesystems bzw. des Mikroskops nicht um einen durch die Fokuslage festgelegten Punkt auszuführen, also nicht um einen solchen Punkt zu pivotieren, sondern um einen Punkt außerhalb der Fokuslage. Beispielsweise in sogenannten Schlüsselloch-Operationsszenarien kann es wünschenswert sein, nicht um einen Punkt auf einer Bodenfläche der Kavität zu pivotieren, da in diesem Fall bereits bei relativ kleinen Drehbewegungen die Sicht durch das Mikroskop durch die das Schlüsselloch begrenzenden Strukturen verdeckt werden kann.

Aktuelle Betriebsszenarien zur Einstellung eines Drehpunkts außerhalb der Fokusebene sind zeitaufwendig und erfordern insbesondere eine Vielzahl von Schritten. So ist beispielsweise bekannt, einen solchen Drehpunkt außerhalb der Fokusebene festzulegen, in dem zunächst ein Bereich am Rand des Schlüssellochs auf Höhe des gewünschten späteren Drehpunkts fokussiert wird. Anschließend wird das Mikroskop in eine gewünschten Lage, also in einer gewünschten Position und mit einer gewünschten Orientierung, positioniert, insbesondere derart, dass die optische Achse durch die vom Schlüsselloch umfasste Fläche verläuft, und dann der vorhergehend erläuterte Point-Lock-Modus aktiviert. Hiernach muss, um den Situs zu betrachten, dann aber noch der Fokus des Mikroskops nachgestellt werden.

Nachteile dieses Verfahrens sind, dass mehrere Schritte nacheinander koordiniert durchgeführt werden müssen, die eine wenig intuitive Bedienung bedingen. Weiter ist es erforderlich, den Point-Lock-Modus aktiviert zu belassen, um den eingestellten Drehpunkt nicht zu verlieren. Bei jeder Unterbrechung der Umpositionierung im Point-Lock-Modus, selbst wenn sich der Drehpunkt nicht ändert, ist es erforderlich, den Drehpunkt erneut einzustellen. Zum Einstellen des Drehpunkts ist es weiter erforderlich, den Fokus zunächst auf den Rand des Schlüssellochs einzustellen, wobei es dann erforderlich ist, dass Mikroskop in der gewünschten Lage zu positionieren, bevor der Point-Lock-Modus aktiviert wird. Das erfordert nicht nur zusätzliche Zeitaufwand, sondern unterbricht den gerade in kritischen Situationen wichtigen Blick auf den Situs beim Umpositionieren. Auch ist bei einem solchen Verfahren eine berührungslose Steuerung, die auch als Hands-free-Bedienung bezeichnet werden kann und z.B. durch Betätigung eines Fußschalters erfolgt, nicht oder nur schwer möglich.

Es stellt sich daher das technische Problem, ein Verfahren zum Betrieb eines Mikroskopiesystems sowie eine Mikroskopiesystems zu schaffen, die eine Bedienung des Mikroskopiesystems vereinfachen.

Die Lösung des technischen Problems ergibt sich durch die Gegenstände mit den Merkmalen der unabhängigen Ansprüche. Weitere vorteilhafte Ausgestaltungen der Erfindung ergeben sich aus den Unteransprüchen.

Vorgeschlagen wird ein Verfahren zum Betreiben eines Mikroskopiesystems.

Das Mikroskopiesystem umfasst ein Mikroskop. Im Sinne dieser Erfindung zeichnet ein Mikroskop eine Einrichtung zur vergrößernden visuellen Darstellung eines Untersuchungsobjekts. Das Mikroskop kann ein klassisches Lichtmikroskop sein, welches ein vergrößertes Abbild durch Ausnutzung optischer Effekte erzeugt, insbesondere durch Mittel zur Strahlführung und/oder -formung und/oder -ablenkung, beispielsweise Linsen. Das Mikroskop kann aber auch ein Digitalmikroskop sein, wobei das von dem Mikroskop zu visualisierende Abbild mittels einer Bilderfassungseinrichtung erzeugt und auf einer entsprechenden Anzeigeeinrichtung, beispielsweise einem Display, zur Anzeige gebracht werden kann.

Das Mikroskop kann insbesondere mindestens ein Okular umfassen. Das Okular bezeichnet einen Teil des Mikroskops, durch den oder in den ein Benutzer schaut, um das vom Mikroskop erzeugte Abbildung visuell zu erfassen.

Weiter kann das Mikroskop mindestens ein Objektiv umfassen. Dieses Objektiv kann eine reelle optische Abbildung eines Untersuchungsobjektes erzeugen. Das Objektiv kann hierbei optische Elemente zur Strahlführung und/oder -formung und/oder -ablenkung umfassen.

Das Mikroskop kann eine optische Achse aufweisen. Diese kann die optische Achse des Objektivs sein. Umfasst das Mikroskop eine stereoskopisches Kamerasystem, so schneiden sich die optischen Achsen der beiden Bilderfassungseinrichtungen des stereoskopischen Kamerasystems im Fokuspunkt. In diesem Fall kann die optische Achse des Mikroskops der Winkelhalbierenden zwischen den optischen Achsen der beiden Bilderfassungseinrichtungen entsprechen, die ebenfalls durch den Fokuspunkt verläuft.

Weiter kann das Mikroskop einen Mikroskopkörper umfassen. Der Mikroskopkörper kann hierbei weitere optische Elemente zur Strahlführung und/oder -formung und/oder -ablenkung umfassen. Es ist möglich, dass das Objektiv lösbar, also auch austauschbar, an dem Mikroskopkörper befestigt ist. Allerdings ist es auch möglich, dass das Objektiv fest in den oder an den Mikroskopkörper integriert ist. Hierbei kann das Objektiv ortsfest relativ zum Mikroskopkörper angeordnet sein.

Weiter kann das Mikroskopiesystem ein Stativ zur Halterung des Mikroskops umfassen. Das Mikroskop, insbesondere der Mikroskopkörper, kann somit an dem Stativ mechanisch befestigt sein. Es ist möglich, dass das Mikroskop an einem freien Ende des Stativs befestigt ist, insbesondere beweglich, z.B. schwenkbar. Das Stativ ist hierbei derart ausgebildet, dass es eine Bewegung des Mikroskops im Raum ermöglicht, insbesondere mit mindestens einem Freiheitsgrad, vorzugsweise mit sechs Freiheitsgraden. Es ist selbstverständlich auch möglich, dass das Stativ derart ausgebildet ist, dass es eine Bewegung des Mikroskops im Raum mit einer beschränkten Anzahl von Freiheitsgraden ermöglicht, insbesondere also mit weniger als sechs Freiheitsgraden.

Ein Freiheitsgrad kann hierbei ein Translations- oder ein Rotationsfreiheitsgrad sein. Insbesondere kann eine Bewegung mit drei voneinander verschiedenen Translationsfreiheitsgraden und drei voneinander verschiedenen Rotationsfreiheitsgraden durch das Stativ ermöglicht werden.

Die Freiheitsgrade können sich hierbei auf ein globales Referenzkoordinatensystem beziehen. Eine Vertikalachse (z-Achse) dieses Referenzkoordinatensystems kann parallel zur Gravitationskraft und entgegengesetzt zu dieser orientiert sein. Eine Längsachse (x-Achse) des Referenzkoordinatensystems und eine Querachse (y-Achse) des Referenzkoordinatensystems können hierbei eine Ebene aufspannen, die senkrecht zur Vertikalachse orientiert ist. Weiter können auch die Längs- und die Querachse orthogonal zueinander orientiert sein. Somit kann das Referenzkoordinatensystem ein kartesisches Koordinatensystem sein.

Weiter kann das Stativ mindestens eine Antriebseinrichtung zur Bewegung des Mikroskops umfassen. Vorzugsweise umfasst das Stativ mehrere Antriebseinrichtungen. Eine Antriebseinrichtung bezeichnet hierbei eine Einrichtung zur Erzeugung einer Antriebskraft bzw. eines Antriebsmoments. Eine solche Antriebseinrichtung kann beispielsweise ein Servomotor sein. Selbstverständlich kann das Stativ auch Mittel zur Kraft-/ Momentenübertragung, z.B. Getriebeeinheiten, umfassen. Insbesondere ist es möglich, die mindestens eine Antriebseinrichtung derart anzusteuern, dass das Mikroskop eine gewünschte Bewegung und somit eine gewünschte Lageänderung im Raum ausführt oder eine gewünschte Lage im Raum einnimmt. Hierbei bezeichnet eine Lage eine Position und/oder eine Orientierung. Es ist hierbei möglich, dass eine Geschwindigkeit der Bewegung auf eine vorbestimmte Maximalgeschwindigkeit beschränkt ist.

Beispielsweise kann die mindestens eine Antriebseinrichtung derart angesteuert werden, dass eine optische Achse des Objektivs eine gewünschte Orientierung einnimmt. Weiter kann die mindestens eine Antriebseinrichtung derart angesteuert werden, dass ein Referenzpunkt des Mikroskops, z.B. ein Fokuspunkt, an einer gewünschten Position im Raum positioniert wird.

Eine Soll-Lage kann hierbei von einem Benutzer oder einem anderen übergeordneten System vorgegeben werden. Verfahren zur Steuerung der mindestens einen Antriebseinrichtung in Abhängigkeit einer Soll-Lage und einer kinematischen Struktur des Stativs sind hierbei dem Fachmann bekannt. Der Benutzer kann hierbei eine Person bezeichnen, die das Mikroskop bedient, insbesondere die in/durch das Okular schaut, um ein Objekt vergrößert zu betrachten. Es ist möglich, dass das Mikroskop ein sogenanntes Operationsmikroskop ist. In diesem Fall kann der Benutzer insbesondere ein Chirurg sein.

Weiter wird ein Drehpunkt festgelegt und das Mikroskopiesystem, insbesondere die mindestens eine Antriebseinrichtung und/oder mindestens eine Bremseinrichtung, des Mikroskopiesystems derart betrieben, dass sich das erläuterte Mikroskop des Mikroskopiesystems, um den festgelegten Drehpunkt herum bewegt, insbesondere aber nicht zwingend mit einem konstanten Abstand. Dieser Drehpunkt kann auch als Pivotpunkt bezeichnet werden. Insbesondere kann das Mikroskopiesystem derart betrieben werden, dass das Mikroskop, z.B. bei einer durch manuelle Betätigung bewirkten Bewegung, um den festgelegten Drehpunkt herum bewegt wird. Dies kann z.B. erreicht werden, indem durch den Betrieb eine entsprechende Bewegungsführung erfolgt bzw. bewirkt wird, z.B. in Form einer kraftgeregelten Bewegungsführung. Allerdings kann das Mikroskopiesystem auch derart betrieben werden, dass die erläuterte Antriebseinrichtung derart gesteuert oder geregelt wird, dass eine durch diese bewirkte Bewegung des Mikroskops entsprechend realisiert wird, z.B. durch eine Positionsregelung.

Eine Bremseinrichtung kann hierbei derart ausgebildet und/oder derart angeordnet sein, dass die Bewegung des Mikroskops um/entlang mindestens einer Achse gebremst wird. Auch kann durch die Bremseinrichtung die Bewegung um/entlang mehrere, aber nicht alle, Achsen gebremst werden. Weiter alternativ kann durch die Bremseinrichtung die Bewegung um/entlang aller Achsen gebremst werden.

Der Drehpunkt wird als Schnittpunkt der optischen Achse des Mikroskops und einer Referenzfläche bestimmt. Hierzu kann, wie nachfolgend noch näher erläutert, eine Lage der optischen Achse bestimmt werden.

Erfindungsgemäß ist die Lage der Referenzfläche fokuslagenunabhängig in einem Referenzkoordinatensystem festgelegt. Mit anderen Worten ist die Lage der Referenzfläche in einem fokuslagenunabhängigen Referenzkoordinatensystem festgelegt. Hierzu kann die Lage, insbesondere vorab, entsprechend festgelegt werden. Weiter wird der Drehpunkt als der Schnittpunkt der optischen Achse mit der derart festgelegten Referenzfläche im Referenzkoordinatensystem bestimmt wird. Dass die Lage fokuslagenunabhängig festgelegt ist bedeutet, dass sich die Lage der Referenzfläche nicht verändert, wenn sich die Fokuslage, z.B. im globalen Referenzkoordinatensystem, verändert. Alternativ, vorzugsweise aber kumulativ, kann die Lage der Referenzfläche objektebenenunabhängig im Referenzkoordinatensystem festgelegt sein. Mit anderen Worten kann die Lage der Referenzfläche in einem objektebenenunabhängigen Referenzkoordinatensystem festgelegt sein. Dass die Lage objektebenenunabhängig festgelegt ist bedeutet, dass sich die Lage der Referenzfläche nicht verändert, wenn sich die Objektebene, insbesondere ihre Lage, z.B. im globalen Referenzkoordinatensystem, verändert. Die Referenzfläche kann insbesondere eine ebene, also ungekrümmte, Fläche sein. Allerdings ist es auch vorstellbar, dass die Referenzfläche eine unebene, z.B. gekrümmte, Fläche ist.

Somit kann Lage der Referenzfläche in einem fokuslagenunabhängigen und einem objektebenenunabhängigen Referenzkoordinatensystem festgelegt sein. Es ist möglich, dass eine Festlegung in einem fokuslagenunabhängigen Referenzkoordinatensystem ebenfalls eine Festlegung in einem objektebenenunabhängigen Referenzkoordinatensystem ist.

Es ist auch möglich, dass die Lage der Referenzfläche in einem schärfeebenenunabhängigen Referenzkoordinatensystem festgelegt ist, insbesondere alternativ oder kumulativ zur Festlegung in einem fokuslagenunabhängigen und/oder objektebenenunabhängigen Referenzkoordinatensystem. Dass die Lage schärfeebenenunabhängig festgelegt ist bedeutet, dass sich die Lage der Referenzfläche nicht verändert, wenn sich die Schärfenebene, insbesondere ihre Lage, z.B. im globalen Referenzkoordinatensystem, verändert. Die Schärfenebene kann eine Ebene im Objektraum bezeichnen, wobei ein Objekt in dieser Ebene mit einer gewünschten Schärfe abgebildet wird. Sie kann orthogonal zur optischen Achse des Mikroskops orientiert sein, die die Schärfenebene in der Mitte des Schärfentiefenbereichs schneidet. Der Schärfentiefenbereich kann in bekannter Weise abhängig von einer aktuell eingestellten Brennweite, der aktuell eingestellten Entfernung als auch der aktuell eingestellten Blendenöffnung sein. Ein Arbeitsabstand des Mikroskops kann einen Abstand zwischen der Schärfeebene und einem Abschlusselement eines Objektivsystems des Mikroskops entlang einer optischen Achse des Mikroskops bezeichnen, die durch das Objektiv bzw. Objektivsystem des Mikroskops festgelegt sein kann. Auch kann der Arbeitsabstand einen Abstand zwischen dem Objektiv, insbesondere dem Abschlusselement, und dem Objekt bei maximaler Fokussierung sein. Der Arbeitsabstand kann eingestellt werden, z.B. durch einen Nutzer oder durch die Durchführung einer Autofokusfunktion, um die Lage der Schärfeebene festzulegen. Verändert sich der Arbeitsabstand, so verändert sich auch die Lage der Schärfeebene. Somit kann eine Veränderung des Fokuspunkts, insbesondere von dessen Lage, auch eine Veränderung der Lage der Schärfeebene bedingen. Die Objektebene kann der Schärfeebene entsprechen.

Die Lage der Referenzfläche im besagten Referenzkoordinatensystem kann hierbei vorbestimmt sein, wobei Informationen über eine Lage und gegebenenfalls weitere referenzflächenspezifische Eigenschaften wie deren Form in einer Speichereinrichtung abrufbar gespeichert sein können. In diesem Fall ist es also erforderlich, die Lage der Referenzfläche im Referenzkoordinatensystem und gegebenenfalls weitere Eigenschaften vor der Bestimmung des Drehpunkts einmalig, insbesondere vor der ersten Bestimmung eines Drehpunkts, zu bestimmen, wobei diese Informationen dann bei einer (Neu-)Bestimmung des Drehpunkts abgerufen und entsprechend ausgewertet werden. Verschiedene Ausführungsformen dieser Bestimmung werden nachfolgend näher erläutert.

Das Referenzkoordinatensystem kann ein patientenfestes oder körperteilfestes Koordinantensystem sein, z.B. ein knochen- oder schädelfestes Koordinatensystem. Auch kann das Koordinatensystem das vorhergehend erläuterte globale Referenzkoordinatensystem sein. Weiter alternativ kann das Referenzkoordinatensystem ein mikroskopiesystem- oder mikroskopfestes Koordinatensystem sein. Beispielsweise kann das Referenzkoordinatensystem ein Koordinatensystem sein, welches ortsfest relativ zu einem Basisabschnitt eines Stativs des Mikroskopiesystems angeordnet ist. An diesem Basisabschnitt können bewegliche Teile des Stativs befestigt sein. Der Basisabschnitt kann bei einem bestimmungsgemäßen Gebrauch ortsfest auf einer Bodenfläche angeordnet sein. Dies schließt nicht aus, dass der Basisabschnitt zum Transport des Mikroskopiesystems entlang der Bodenfläche bewegt werden kann, z.B. auf Rollen.

Somit wird der Drehpunkt derart bestimmt, dass sich der Drehpunkt, insbesondere zeitlich nach der Festlegung des Drehpunkts, beim Verändern einer Fokuslage des Mikroskops nicht verändert. Mit anderen Worten ändert sich also die Lage des festgelegten Drehpunkts nicht, wenn nach der Festlegung des Drehpunkts die Fokuslage des Mikroskops verändert wird. Es ist auch möglich, dass sich die Lage des festgelegten Drehpunkts nicht verändert, wenn nach der Festlegung des Drehpunkts die Lage der Objektebene und/oder der Schärfeebene des Mikroskops verändert wird.

Insbesondere kann die Referenzfläche derart bestimmt werden, dass sie nicht der Fokusebene entspricht oder nicht vollständig in der Fokusebene des Mikroskops angeordnet ist. Es ist aber vorstellbar, dass ein Teil der Referenzfläche in der Fokusebene angeordnet ist oder diese schneidet. Auch kann die Referenzfläche derart bestimmt werden, dass sie nicht der Objektebene und/oder Schärfeebene entspricht oder nicht vollständig in der dieser/diesen Ebene(n) angeordnet ist. Es ist aber vorstellbar, dass ein Teil der Referenzfläche in dieser/diesen Ebene(n) angeordnet ist oder diese schneidet.

Die Fokusebene bezeichnet hierbei eine Ebene, in der der Fokuspunkt angeordnet ist und die senkrecht zur optischen Achse verläuft. Somit kann mit dem vorgeschlagenen Verfahren als Drehpunkt nicht der Fokuspunkt, sondern vielmehr ein Drehpunkt außerhalb der Fokusebene festgelegt werden.

Auch ist es möglich, den Drehpunkt unabhängig vom Fokuspunkt festzulegen. Dies kann bedeuten, dass der Drehpunkt derart festgelegt wird, dass er sich bei Änderung des Fokuspunkts nicht ändert.

Die Referenzfläche kann hierbei datenbasiert bestimmt werden. Dies kann bedeuten, dass die Referenzfläche basierend auf präoperativ oder intraoperativ erzeugten Daten bestimmt wird. Intraoperativ erzeugte Daten können insbesondere Daten sein, die topografische Informationen des Situs kodieren. Auch können intraoperativ erzeugte Daten solche Daten sein, die von einer Lageerfassungseinrichtung erzeugt werden und die eine Raumlage eines Lagemarkierungsinstruments oder eines Markers kodieren. Dies wird nachfolgend noch näher erläutert. Auch können die Daten Betriebsparameter des Mikroskops kodieren.

Auch kann die Referenzfläche basierend auf Daten einer Nutzereingabe bestimmt werden.

Durch die Festlegung des Drehpunkts als Schnittpunkt der optischen Achse mit einer Referenzfläche ergibt sich in vorteilhafter Weise eine einfache, genaue und zuverlässige Festlegung des Drehpunkts. Weiter kann in vorteilhafter Weise erreicht werden, dass eine einfache, genaue und zuverlässige Festlegung eines Drehpunkts außerhalb der Fokusebene gewährleistet wird. Dies wiederum verbessert in vorteilhafter Weise die Bedienung des Mikroskopiesystems, insbesondere bei dem vorhergehend erläuterten Schlüsselloch-Operationsszenario, wobei der Drehpunkt beispielsweise, wie nachfolgend noch näher erläutert, als ein Punkt in der von einer Situsöffnung umfassten Fläche festgelegt werden kann. Im Vergleich zu bisherigen Verfahren ergibt sich somit eine einfache und somit auch zeitsparende Bedienung des Mikroskopiesystems, insbesondere in einem solchen Operationsszenario.

Der Referenzfläche zur Bestimmung des Drehpunkts muss hierbei in vorteilhafter Weise nur einmalig festgelegt werden und kann dann in abrufbarer Form abgespeichert werden. Dann werden erfindungsgemäß zu einem späteren Zeitpunkt abgespeicherte Informationen über die Referenzfläche abgerufen und ein neuer Drehpunkt als Schnittpunkt der optischen Achse in ihrer aktuellen Lage mit der abgespeicherten Referenzfläche bestimmt. Dann kann die mindestens eine Antriebseinrichtung und/oder mindestens eine Bremseinrichtung, des Mikroskopiesystems derart betrieben werden, dass sich das erläuterte Mikroskop des Mikroskopiesystems, um den neu festgelegten Drehpunkt herum bewegt. Hierdurch wird ermöglicht, dass ein wiederholtes Bewegen des Mikroskops um einen Drehpunkt in der Referenzfläche herum, welches auch als Pivotieren bezeichnet werden kann, ohne erneute Bestimmung und Festlegung der Referenzfläche ermöglicht wird. Dies ermöglicht, dass ein Nutzer einen Pivotier-Modus wiederholt aktivieren kann, wobei die Drehbewegung dann immer um einen Drehpunkt in der gespeicherten Referenzfläche ausgeführt wird.

In einer weiteren Ausführungsform ist die Lage der Referenzfläche in einem objektebenenunabhängigen Referenzkoordinatensystem festgelegt. Dies und entsprechende Vorteile wurden vorhergehend bereits erläutert.

In einer weiteren Ausführungsform wird bzw. ist die Lage und/oder die Form der Referenzfläche derart festgelegt, dass die von einer Situsöffnung umfasste Fläche die Referenzfläche bildet oder von der Referenzfläche umfasst wird.

Die Situsöffnung kann durch anatomische Strukturen festgelegt sein. So kann die Situsöffnung insbesondere eine Schädelöffnung sein, wobei diese Öffnung durch den die Öffnung umgebenen Schädel festgelegt ist. Es ist auch vorstellbar, dass die Situsöffnung durch medizinische Instrumente, beispielsweise einen Trokar oder durch medizinische Klammern, festgelegt ist.

Die von der Situsöffnung umfasste Fläche kann hierbei eine Fläche bezeichnen, die von einem Rand der Situsöffnung in einer Querschnittsebene begrenzt wird, wobei diese Querschnittsebene senkrecht zu einer zentralen Mittellinie der Situsöffnung orientiert ist. Mit anderen Worten wird die Fläche in der genannten Querschnittsebene von dem Rand der Situsöffnung begrenzt.

Für verschiedene Querschnittsebenen entlang der zentralen Mittellinie kann sich eine Form und/oder Größe der umfassten Fläche verändern. In diesem Fall kann die umfasste Fläche beispielsweise als die Fläche mit minimalster Größe aus der Menge der sich für verschiedene Querschnittsebenen ergebenden Flächen bestimmt werden.

Hierdurch ergibt sich in vorteilhafter Weise, dass einfach, schnell, genau und zuverlässig ein Drehpunkt für das Mikroskopiesystems unabhängig von, insbesondere außerhalb, der Fokusebene derart festgelegt werden kann, dass ein Nutzer bei einem Betrieb des Mikroskopiesystems mit diesem festgelegten Drehpunkt, also in einem mit diesem Drehpunkt aktivierten Point-Lock-Modus, zuverlässig durch die Situsöffnung hindurch schauen kann, ohne dass beim Verändern des Fokus die Sicht verdeckt wird. Auch wird eine vereinfachte Festlegung des Drehpunkts ermöglicht. Dies ist insbesondere bei den bereits erläuterten Schlüsselloch-Operationsszenarien von Vorteil.

In einer weiteren Ausführungsform wird die Situsöffnung, insbesondere der Form und/oder deren Lage, automatisch festgelegt. Hierzu können insbesondere Ausgangssignale von Erfassungseinrichtungen, insbesondere Sensoren, ausgewertet werden. Beispielhafte Verfahren hierzu werden nachfolgend noch näher erläutert. Auch ist es möglich, die Situsöffnung durch Auswertung von präoperativ oder intraoperativ erzeugten Daten, insbesondere Bilddaten, festzulegen.

Alternativ wird die Situsöffnung manuell, insbesondere durch eine Nutzereingabe, festgelegt.

Selbstverständlich ist es auch möglich, dass die Situsöffnung semiautomatisch festgelegt wird, wobei ein automatischer Algorithmus zur Festlegung durch Nutzereingaben unterstützt wird.

Daten zur Bestimmung der Referenzfläche werden in diesem Fall also z.B. durch Erfassungseinrichtungen oder durch eine Nutzereingabe erzeugt.

Hierdurch ergibt sich in vorteilhafter Weise eine einfache und zuverlässige Festlegung der Situsöffnung und somit der Referenzfläche.

In einer weiteren Ausführungsform ist die Referenzfläche eine begrenzte Fläche. Es ist möglich, dass die Referenzfläche eine allseitig begrenzte Fläche ist. In diesem Fall kann die Referenzfläche beispielsweise eine kreisförmige Fläche oder eine ovale Fläche sein. Selbstverständlich ist auch vorstellbar, dass die Referenzfläche andere Formen, insbesondere auch eine Freiform, aufweist.

Alternativ ist es möglich, dass die Referenzfläche eine nicht allseitig begrenzte Fläche ist. Beispielsweise kann die Referenzfläche eine nur einseitig begrenzte Fläche sein. In diesem Fall kann es möglich sein, dass zumindest ein Abschnitt eines Randes der Referenzfläche Teil kreisförmig oder Teil ovalförmig ist. Selbstverständlich ist es auch vorstellbar, dass der Abschnitt des Randes eine andere Formen, insbesondere auch eine Freiform, aufweist.

Durch die Verwendung einer Referenzfläche als begrenzte Fläche ergibt sich in vorteilhafter Weise, dass der Drehpunkt zuverlässig in einem gewünschten Bereich festgelegt werden kann, wodurch beispielsweise sichergestellt werden kann, dass der Nutzer bei den Betrieb des Mikroskopiesystems mit dem derart festgelegten Drehpunkt zuverlässig durch die Situsöffnung hindurch schauen kann, insbesondere wenn die Referenzfläche der von der Situsöffnung umfassten Fläche entspricht.

Es ist selbstverständlich auch vorstellbar, dass die Referenzfläche eine unbegrenzte Fläche ist, also eine Ebene. In diesem Fall ergibt sich in vorteilhafter Weise eine besonders einfache Festlegung des Drehpunkts.

In einer weiteren Ausführungsform ist die Referenzfläche eine gekrümmte Fläche. Hierdurch ergibt sich in vorteilhafter Weise eine weitere Verbesserung der Zuverlässigkeit der Festlegung des Drehpunkts, wodurch sich wiederum, wie vorhergehend bereits erläutert, bei einem Betrieb mit diesem festgelegten Drehpunkt das Risiko für einen Sichtverlust durch den Nutzer verringert wird.

In einer weiteren Ausführungsform wird bzw. ist die Lage und/oder die Form der Referenzfläche in Abhängigkeit von präoperativ erzeugten Daten bestimmt. Derart präoperativ erzeugten Daten können beispielsweise CT-basierte Daten oder MRT-basierte Daten seien. Selbstverständlich ist es auch möglich, durch andere Verfahren präoperativ Daten zu erzeugen, die die Lage und/oder die Form der Referenzfläche kodieren oder auf deren Basis die Lage und oder die Form der Referenzfläche festgelegt werden kann.

Beispielsweise kann bei einer Operationsplanung auf Grundlage von präoperativ erzeugten Daten, insbesondere die Anatomie des zu operierenden Patienten repräsentierenden Daten, die Lage und/oder die Form einer Situsöffnung festgelegt werden.

Hierbei kann es erforderlich sein, die präoperativen Daten in einen geometrischen Zusammenhang mit dem erläuterten Referenzkoordinatensystem zu bringen. Dies kann beispielsweise durch einen dem Fachmann bekannten Registrierungsvorgang erfolgen. Dieser ermöglicht insbesondere die Bestimmung einer Transformationsvorschrift zur Transformation der auf Grundlage der präoperativ erzeugten Daten bestimmten Lage und/oder Form von dem Koordinatensystem der präoperativen Daten in das Referenzkoordinatensystem. Diese kann genutzt werden, um die Lage und/oder die Form der Referenzfläche in Abhängigkeit der präoperativ erzeugten Daten zu bestimmen.

Dadurch ergibt sich in vorteilhafter Weise eine einfache Bestimmung der Lage und oder der Form der Referenzfläche.

In einer weiteren Ausführungsform werden topografische Informationen des Situs bestimmt, wobei die Lage und/oder Form der Referenzfläche in Abhängigkeit dieser Informationen bestimmt wird. Die zur Bestimmung der Referenzfläche verwendeten Daten können hierbei die topografischen Informationen oder einen Teil davon kodieren.

Die topografischen Informationen können durch eine Einrichtung zur Erzeugung topografischer Informationen erzeugt werden. Eine solche Einrichtung kann insbesondere eine Bilderfassungseinrichtung umfassen. In diesem Fall kann also die Lage und/oder die Form der Referenzfläche bildbasiert bestimmt werden. Hierzu können von mindestens einer Bilderfassungseinrichtung erzeugte Abbilder ausgewertet werden. Eine Bilderfassungseinrichtung kann hierbei eine Einrichtung zur Bestimmung von topografischen Informationen des Situs bezeichnen. Eine Bilderfassungseinrichtung kann ein zwei- oder dreidimensionales Abbild erzeugen und beispielsweise eine CCD-Kamera oder eine CMOS-Kamera sein. Allerdings kann eine Bilderfassungseinrichtung auch eine Time-of-flight-Kamera sein. Die Auswertung kann durch eine Auswerteeinrichtung erfolgen, die als Mikrocontroller oder integrierte Schaltung ausgebildet sein oder eine(n) solche(n) umfassen kann.

Insbesondere kann/können hierzu mindestens ein, vorzugsweise mehrere, von dem Mikroskopiesystem erzeugte(s) Bild(er) ausgewertet werden. In diesem Fall kann das Mikroskopiesystems mindestens eine Bilderfassungseinrichtung umfassen, deren Abbilder zur Bestimmung der Lage und/oder der Form der Referenzfläche ausgewertet werden, insbesondere durch eine Auswerteeinrichtung des Mikroskopiesystems.

Die Bilderfassungseinrichtung des Mikroskopiesystems kann insbesondere die Bilderfassungseinrichtung sein, die zu Digitalisierung des vom Mikroskop erzeugten Abbilds dient, wobei dieses dann z.B. auf einer entsprechenden Anzeigeeinrichtung des Mikroskopiesystems dargestellt wird.

Alternativ können auch Abbilder einer Bilderfassungseinrichtung ausgewertet werden, welche nicht Teil des Mikroskopiesystems ist und/oder nicht zur erläuterten Digitalisierung dient. Beispielsweise kann die Bilderfassungseinrichtung eine Bilderfassungseinrichtung einer Lageerfassungseinrichtung sein. Die Lageerfassungseinrichtung kann eine Lageerfassungseinrichtung des Mikroskopiesystems sein. In diesem Fall kann z.B. die Bilderfassungseinrichtung am Mikroskopiesystems angeordnet/befestigt sein, wobei sie jedoch nicht zu Digitalisierung des vom Mikroskop erzeugten Abbilds dient.

Zur bildbasierten Bestimmung der Lage und/oder der Form können dem Fachmann bekannte Verfahren der Bildverarbeitung angewendet werden, beispielsweise Segmentierungsverfahren. Wie im Falle der Verwendung präoperativer Daten kann es erforderlich sein, durch eine Registrierung einen geometrischen Zusammenhang zwischen einem Koordinatensystem der Bilderfassungseinrichtung und dem vorhergehend erläuterten Referenzkoordinatensystem zu bestimmen, wobei dann die Form und/oder die Lage in Abhängigkeit dieses geometrischen Zusammenhangs, beispielsweise in Form einer Koordinatentransformation, bestimmt werden kann.

Es ist beispielsweise möglich, dass in einem oder mehreren Abbild(er) eine Situsöffnung detektiert wird, beispielsweise durch ein geeignetes Segmentierungsverfahren. Dann kann als Referenzfläche die von dem Rand der Situsöffnung umfasste Fläche festgelegt werden.

Es ist weiter möglich, dass die Lage und/oder Form der Referenzfläche in Abhängigkeit von topografischen Informationen und zusätzlich in Abhängigkeit einer Blickrichtung des Mikroskops und/oder einer Blickrichtung eines Nutzers bestimmt wird.

Hierzu kann eine Blickrichtung des Mikroskops bestimmt werden. Dies kann beispielsweise in Abhängigkeit der Ausrichtung des Mikroskops bestimmt werden. Beispielsweise kann die Blickrichtung als die Richtung/Orientierung der optischen Achse, insbesondere im Referenzkoordinatensystem, bestimmt werden.

Die Blickrichtung des Nutzers kann der Blickrichtung des Mikroskops entsprechen. Alternativ kann die Blickrichtung des Nutzers durch eine Einrichtung zur Blickrichtungserkennung bestimmt werden, beispielsweise ein dem Fachmann bekanntes Gaze-Tracking-System.

Beispielsweise kann ein Schnittpunkt zwischen der Blickrichtung und einer in Abhängigkeit der topographischen Informationen bestimmbaren Oberfläche des Situs bestimmt werden. Die Referenzfläche kann dann derart festgelegt werden, dass dieser Schnittpunkt in der Referenzfläche angeordnet ist. Auch kann in Abhängigkeit der Lage dieses Schnittpunkts dann ein Suchbereich für eine Situsöffnung bestimmt werden, wobei dann durch geeignete Verfahren zur Auswertung topografischer Informationen, beispielsweise zur Bildverarbeitung, dann eine Situsöffnung gesucht wird. Werden mehrere Situsöffnungen detektiert, so kann als Referenzfläche die von dem Rand der dem Schnittpunkt am nächsten liegende Situsöffnung umfasste Fläche festgelegt werden. Auch ist es möglich, dass ein Nutzer die Blickrichtung des Mikroskops oder seine Blickrichtung auf den Rand der Referenzfläche ausrichtet und in einem vorbestimmten Zeitintervall derart verändert, dass diese entlang des Rand bewegt wird, und damit die Begrenzung der Referenzfläche festlegt.

Es ist weiter möglich, dass die Lage und/oder Form der Referenzfläche in Abhängigkeit von topografischen Informationen und weiter zusätzlich in Abhängigkeit einer Lage eines Lagemarkierungsinstruments und/oder eines Markers bestimmt wird. Ein Lagemarkierungsinstrument und ein Marker werden nachfolgend noch näher erläutert.

Hierbei die Lage und/oder Form durch Auswertung der topografischen Informationen, insbesondere also bildbasiert, detektiert werden.

Weiter kann mindestens ein Marker derart angeordnet sein, dass in Abhängigkeit der Lage des Markers die Lage und/oder Form der Referenzfläche bestimmt werden kann. So können z.B. drei oder mehr als drei Marker derart angeordnet sein, dass deren Position in einer gemeinsamen Ebene liegt, wobei in dieser Ebene auch die Referenzfläche angeordnet ist. Verbindungslinien zwischen Markern können eine Begrenzungslinie der Referenzfläche kodieren. Auch kann die Orientierung eines Markers eine Orientierung der Referenzfläche kodieren. Somit ist es auch möglich, dass durch die Lage eines einzelnen Markers die Lage der Referenzfläche festgelegt ist.

Auch kann in Abhängigkeit der Lage des Instruments/des Markers ein Suchbereich für eine Situsöffnung bestimmt werden, wobei dann durch geeignete Verfahren zur Auswertung topografischer Informationen, beispielsweise zur Bildverarbeitung, dann eine Situsöffnung gesucht wird. Werden mehrere Situsöffnungen detektiert, so kann als Referenzfläche die von dem Rand der der Lage des Instruments/des Markers am nächsten liegende Situsöffnung umfasste Fläche festgelegt werden.

Hierdurch ergibt sich in vorteilhafter Weise ebenfalls eine einfache und zuverlässige Bestimmung der Form und und/oder der Lage der Referenzfläche.

In einer bevorzugten Ausführungsform wird die Referenzfläche bestimmt, indem mindestens ein Referenzflächenpunkt festgelegt wird, wobei die Referenzfläche als Ebene festgelegt wird oder in der Ebene angeordnet ist, die senkrecht zur optischen Achse des Mikroskops orientiert ist und in der der Referenzflächenpunkt angeordnet ist. Hierbei können die zur Bestimmung verwendeten Daten die Lage des Referenzflächenpunkts und die Orientierung der optischen Achse umfassen bzw. Informationen hierüber kodieren.

Hierzu kann es erforderlich sein, eine Orientierung der optischen Achse des Mikroskops zu bestimmen, insbesondere im vorhergehend erläuterten Referenzkoordinatensystem. Weiter kann es erforderlich sein, die Lage des Referenzflächenpunkts festzulegen, vorzugsweise ebenfalls im vorhergehend erläuterten Referenzkoordinatensystem. Beispielhafte Verfahren zur Festlegung der Lage des Referenzflächenpunkt werden nachfolgend noch erläutert.

Der Referenzflächenpunkt entspricht hierbei nicht bzw. nicht zwingend dem vorhergehend erläuterten Drehpunkt.

Hierdurch ergibt sich in vorteilhafter Weise eine einfache und schnelle Festlegung der Referenzfläche.

Wird die Referenzfläche derart bestimmt, so kann der Referenzflächenpunkt nur einmalig festgelegt und beispielsweise in abrufbarer Form abgespeichert werden. Dann können zu einem späteren Zeitpunkt abgespeicherte Informationen über den Referenzflächenpunkt abgerufen und die Referenzfläche in Abhängigkeit des Referenzflächenpunkts bestimmt werden, wobei ein neuer Drehpunkt als Schnittpunkt der optischen Achse in ihrer aktuellen Lage mit der derart bestimmten Referenzfläche bestimmt wird. Dann kann die mindestens eine Antriebseinrichtung und/oder mindestens eine Bremseinrichtung, des Mikroskopiesystems derart betrieben werden, dass sich das erläuterte Mikroskop des Mikroskopiesystems, um den neu festgelegten Drehpunkt herum bewegt. Hierdurch wird ermöglicht, dass ein wiederholtes Bewegen des Mikroskops um einen Drehpunkt in der Referenzfläche herum, welches auch als Pivotieren bezeichnet werden kann, ohne erneute Festlegung des Referenzflächenpunkts ermöglicht wird.

Insbesondere, aber nicht ausschließlich, in dieser Ausführungsform kann die Referenzfläche eine vorbestimmte geometrische Form aufweisen, beispielsweise eine Kreisform oder eine ovale Form, wobei Eigenschaften der geometrischen Form vorbestimmt sein können.

In einer weiteren Ausführungsform wird der mindestens eine Referenzflächenpunkt als von einem Nutzer eingestellter Fokuspunkt bestimmt. In diesem Fall können Daten zur Bestimmung der Referenzfläche also zusätzlich Betriebsparameterdaten des Mikroskops umfassen.

Hierbei kann nach der Bestimmung/Festlegung des Referenzflächenpunkts die Fokuslage des Mikroskopiesystems wieder verändert werden, wobei jedoch die Referenzfläche in Abhängigkeit des vor der Änderung eingestellten Referenzflächenpunkts bestimmt wird. Wird eine Lage der Referenzfläche bzw. Referenzflächenpunkts während einer Operation bzw. einer Operationsphase nicht verändert, so ist muss der Referenzflächenpunkt nur einmalig festgelegt werden. Veränderungen der Lage der Referenzfläche bzw. Referenzflächenpunkts können sich beispielsweise durch Lageänderungen des Patienten ergeben.

Ist eine Änderung der Lage der Referenzfläche bzw. Referenzflächenpunkts während einer Operation bzw. einer Operationsphase bestimmbar, beispielsweise durch ein geeignetes Lageverfolgungssystem, so kann auch die Lage der Referenzfläche bzw. des Referenzflächenpunkts entsprechend der bestimmten Lageänderung aktualisiert werden.

Alternativ wird der mindestens eine Referenzflächenpunkt durch die Positionierung eines Lagemarkierungsinstruments festgelegt. Das Lagemarkierungsinstrument kann insbesondere ein von einem Nutzer, insbesondere während einer Operation, manuell positionierbares Instrument sein. Es ist beispielsweise möglich, den Referenzflächenpunkt festzulegen, in dem das Lagemarkierungsinstruments von einem Nutzer in einer der gewünschten Lage des Referenzflächenpunkt entsprechenden Lage positioniert wird, wobei dann eine Lagebestimmungssignal erzeugt wird, beispielsweise durch einen Nutzer. Dieses Signal kann insbesondere durch eine Nutzereingabe, beispielsweise eine haptische, eine akustische oder in einer anderen Form vorgenommenen Nutzereingabe, erzeugt werden. Die Lage des Referenzflächenpunkts kann als die dann bestimmte Lage des Lagemarkierungsinstruments festgelegt werden. Die Lage des Lagemarkierungsinstruments kann beispielsweise der Lage eines vorbestimmten Punktes des Lagemarkierungsinstruments, beispielsweise der Spitze, entsprechen.

Alternativ kann der Referenzflächenpunkt in Abhängigkeit einer Lage mindestens eines Markers bestimmt werden. Ein Marker kann, insbesondere während einer Operation, ein statisch angeordnetes Instrument sein, weiter insbesondere am Situs angeordnet.

Hierzu kann eine Lage des Lagemarkierungsinstruments oder des Markers bestimmt werden, insbesondere im vorhergehend erläuterten Referenzkoordinatensystem. Die Lage kann beispielsweise durch eine Lageerfassungseinrichtung bestimmt werden, beispielsweise eine optische Lageerfassungseinrichtung, insbesondere eine stereoskopische Lageerfassungseinrichtung. Selbstverständlich können auch andere Lageerfassungseinrichtung in verwendet werden.

Auch kann die Lage des Lagemarkierungsinstruments oder des Markers bildbasiert bestimmt werden, beispielsweise in Abhängigkeit von Abbildern einer optischen Lageerfassungseinrichtung oder von Abbildern einer Bilderfassungseinrichtung des Mikroskopiesystems.

Das Lagemarkierungsinstruments oder der Marker kann weiter beispielsweise mindestens ein Target umfassen, wobei dieses mindestens ein Markerelement umfasst oder aufweist. Ein Markerelement kann hierbei insbesondere optisch erfassbar und somit auch in einem Abbild detektierbar sein, insbesondere ein optisch erfassbares Muster aufweisen.

Das Target kann hierbei zum Beispiel an dem Lagemarkierungsinstrument oder dem Marker befestigt sein.

Ein Lagemarkierungsinstrument kann z.B. als Spritze, Pinzette, Löffel, Schere, Skalpell, Zange, Sauger oder Kauter oder anderes, von einem Nutzer während einer Operation dynamisch positioniertes oder positionierbares Instrument ausgebildet sein.

Ein Marker kann beispielsweise als Klemme, insbesondere Hautklemme, Halter, Trokar, Wundhaken, Hautklammergerät, aber auch Retraktor, z.B. einen Gehirn-Retraktor, oder ein anderes, während einer Operation statisch angeordnetes Instrument, ausgebildet sein.

Das Lagemarkierungsinstrument oder der Marker können somit durch eines der erläuterten Instrumente ausgebildet sein.

In diesem Fall können Daten zur Bestimmung der Referenzfläche also von einer Lageerfassungseinrichtung erzeugte Daten sein.

Hierdurch ergibt sich in vorteilhafter Weise ebenfalls eine einfache Festlegung des Referenzflächenpunkt.

Alternativ kann der mindestens eine Referenzflächenpunkt in Abhängigkeit einer Blickrichtungserkennung festgelegt werden. Die Blickrichtungserkennung wurde vorhergehend bereits erläutert.

Weiter kann der Referenzflächenpunkt als ein Punkt entlang der Blickrichtung festgelegt werden, zum Beispiel als Schnittpunkt der Blickrichtungsachse mit einem vorbestimmten oder von einem Nutzer festgelegten topografischen Merkmal. Diese Festlegung kann durch eine Nutzereingabe erfolgen, insbesondere eine akustisch Nutzereingabe in Form eines Sprachkommandos.

Zum Beispiel kann eine Autofokusfunktion ausgeführt werden, wobei als Referenzflächenpunkt dann als der nach der ausgeführten Autofokusfunktion bestimmte Fokuspunkt festgelegt wird. Hierbei kann nach der Bestimmung/Festlegung des Referenzflächenpunkts die Fokuslage des Mikroskopiesystems wieder verändert werden. Hierdurch ergibt sich in vorteilhafter Weise ebenfalls eine einfache Festlegung des Referenzflächenpunkt.

Einer weiteren Ausführungsform wird die Lage eines Referenzflächenpunkts festgelegt, in dem die Lage eines bereits festgelegten Referenzflächenpunkts geändert wird. Beispielsweise kann die Lage geändert werden, indem eine Distanz bestimmt wird, wobei die Lage des Referenzflächenpunkts bestimmt wird, indem die Lage des bereits festgelegten Referenzflächenpunkts um die derart bestimmte Distanz verändert wird, insbesondere entlang einer vorbestimmten Richtung, beispielsweise entlang einer normalen Richtung zur bereits festgelegten Referenzfläche oder entlang der optischen Achse, insbesondere hin zum Mikroskop.

Die Distanz kann beispielsweise durch eine Nutzereingabe, beispielsweise eine haptische, eine akustische oder in einer anderen Form vorgenommenen Nutzereingabe, festgelegt werden. Hierdurch ergibt sich in vorteilhafter Weise eine einfache Änderung des Referenzflächenpunkts und somit auch der Referenzfläche.

In einer weiteren Ausführungsform werden mindestens drei Referenzflächenpunkte bestimmt, wobei die Lage und/oder die Form der Referenzfläche derart bestimmt wird, dass die mindestens drei Referenzflächenpunkte in der Referenzfläche liegen oder ein Abstand der Referenzflächenpunkte von der Referenzfläche oder einer Ebene, die die Referenzfläche enthält, minimal ist. Vorstellbar ist zum Beispiel, dass die Lage und/oder die Form der Referenzfläche derart bestimmt wird, dass die mindestens drei Referenzflächenpunkte Randpunkte der Referenzfläche bilden. Dies ist jedoch nicht zwingend. So ist es auch vorstellbar, dass die mindestens drei Referenzflächenpunkte keine Randpunkte der Referenzfläche bilden. Bezüglich der Festlegung von Referenzflächenpunkt wird auf die vorhergehend erläuterten beispielhaften Verfahren verwiesen. Hierdurch ergibt sich in vorteilhafter Weise ebenfalls eine einfache Festlegung der Referenzfläche.

In einer weiteren Ausführungsform wird als Drehpunkt der Fokuspunkt festgelegt, falls die optische Achse die begrenzte Referenzfläche nicht schneidet. Hierdurch ergibt sich in vorteilhafter Weise, dass ein Nutzer das Mikroskopiesystem in einfacher Weise sowohl in den vorhergehend erläuterten Point-Lock-Modus als auch in einen Modus zur Ausführung einer Bewegung um einen entsprechend des vorgeschlagenen Verfahrens festgelegten Punkt versetzt werden kann.

Es ist möglich, dass das Mikroskopiesystem derart betrieben wird, dass sich ein Mikroskop des Mikroskopiesystems mit einem konstanten Abstand um den Drehpunkt herum bewegt. Eine solche Bewegung mit konstantem Abstand ist insbesondere interessant, wenn eine Bewegung des Mikroskops mittels einer manuell bedienbaren Steuereinrichtung, z.B. einem Joystick, gesteuert wird, wobei die Veränderung des Abstands durch Bedienung der Steuereinrichtung nicht gewünscht oder nicht möglich ist. Der konstante Abstand ermöglicht eine Bewegung des Mikroskops, wobei die optischen Eigenschaften wie z.B. Vergrößerung, Bildausschnitt, Helligkeit und gegebenenfalls weitere Eigenschaften während der Bewegung gleich bleiben bzw. nicht verändert werden müssen. Dies kann natürlich auch sichergestellt werden, wenn der konstante Abstand bei einer durch manuelle Betätigung bewirkten Bewegung gewährleistet wird.

In einer weiteren Ausführungsform wird eine Fokuslage in Abhängigkeit der Änderung des Abstands des Mikroskops vom Drehpunkt geändert, insbesondere also, wenn sich bei einer Bewegung des Mikroskops um den Drehpunkt herum der Abstand ändert. Dies kann automatisiert erfolgen. Der Abstand kann ein Abstand entlang der optischen Achse des Mikroskops sein. Beispielsweise kann die Veränderung der Fokuslage gleich der Veränderung des Abstands sein. Mit anderen Worte kann eine Nachfokussierung durchgeführt werden, sodass ein fokussierter Punkt/Bereich auch bei einer Abstandsänderung weiterhin fokussiert ist. In diesem Fall kann die Fokuslage auch bei einer Bewegung um den Drehpunkt herum im Referenzkoordinatensystem konstant bleiben.

Die Durchführung einer solchen Nachfokussierung ist jedoch nicht zwingend. So kann es erwünscht sein, dass die Fokuslage nicht (automatisiert) in Abhängigkeit der Änderung des Abstands des Mikroskops vom Drehpunkt verändert wird. In diesem Fall kann sich die Fokuslage bei einer Bewegung um den Drehpunkt herum im Referenzkoordinatensystem ändern.

Weiter vorgeschlagen wird ein Mikroskopiesystem, umfassend:
- ein Mikroskop,
- ein Stativ zur Halterung des Mikroskops, wobei das Stativ mindestens eine Antriebseinrichtung zur Bewegung des Mikroskops umfasst,
- mindestens eine Steuer- und Auswerteeinrichtung, wobei das Mikroskopiesystems derart betreibbar, insbesondere durch die Steuer- und Auswerteeinrichtung steuerbar, ist, dass sich das Mikroskop um den Drehpunkt herum bewegt, wobei eine Referenzfläche bestimmt wird, wobei als Drehpunkt ein Schnittpunkt einer optischen Achse des Mikroskops mit der Referenzfläche verwendet wird.

Erfindungsgemäß ist die Lage der Referenzfläche in einem fokuslagenunabhängigen Referenzkoordinatensystem festgelegt und der Drehpunkt als der Schnittpunkt der optischen Achse mit der derart festgelegten Referenzfläche im Referenzkoordinatensystem bestimmbar.

Insbesondere kann das Mikroskopiesystem eine Speichereinrichtung, beispielsweise eine RAM- oder ROM-basierte Speichereinrichtung, umfassen, in der Informationen über die Lage der Referenzfläche im Referenzkoordinatensystem und gegebenenfalls weitere referenzflächenspezifischen Informationen wie z.B. die Form der Referenzfläche abrufbar gespeichert sind. Diese Informationen können durch die Steuer- und Auswerteeinrichtung abgerufen werden, wobei dann durch diese Steuer- und Auswerteeinrichtung auch der Drehpunkt bestimmt werden kann.

Das Mikroskopiesystem ist somit insbesondere derart konfiguriert, dass ein Verfahren gemäß einer der in dieser Offenbarung offenbarten Ausführungsformen mit den Mikroskopiesystems durchführbar ist. Somit ergibt sich in vorteilhafter Weise ein Mikroskopiesystem, mit dem ein solches Verfahren durchführbar ist.

Die Steuer- und Auswerteeinrichtung kann hierbei als Mikrocontroller oder integrierte Schaltung ausgebildet sein oder eine/n solche/n umfassen. Die Steuer- und Auswerteeinrichtung kann insbesondere die vorhergehend erläuterte Auswerteeinrichtung zur Auswertung der topografischen Informationen bilden. Weiter kann die Steuer- und Auswerteeinrichtung die vorhergehend erläuterten Verfahren zur Bestimmung/Festlegung der Referenzfläche durchführen.

In einer weiteren Ausführungsform ist die Lage der Referenzfläche in einem objektebenenunabhängigen Referenzkoordinatensystem festgelegt. Dies und entsprechende Vorteile wurden vorhergehend bereits erläutert.

In einer weiteren Ausführungsform umfasst das Mikroskopiesystem eine Einrichtung zur Erzeugung topografischer Informationen, wobei diese insbesondere als Bilderfassungseinrichtung ausgebildet oder mindestens eine Bilderfassungseinrichtung umfassen kann. Dies und entsprechende Vorteile wurden vorhergehend bereits erläutert. Weiter alternativ oder kumulativ umfasst das Mikroskopiesystem mindestens eine Lageerfassungseinrichtung zur Erfassung einer Lage eines Lagemarkierungsinstruments und/oder eines Markers.

Weiter kann das Mikroskopiesystem eine Einrichtung zur Blickrichtungserkennung eines Nutzers umfassen. Weiter kann das Mikroskopiesystem eine Einrichtung zur Bestimmung einer Lage des Mikroskops, insbesondere einer Blickrichtung des Mikroskops bzw. einer Orientierung der optischen Achse, umfassen.

Ebenfalls kann das Mikroskopiesystem eine Einrichtung zur Nutzereingabe umfassen. Weiter kann das Mikroskopiesystem eine Schnittstelle zum Einlesen von präoperativen Daten umfassen.

Die Erfindung wird anhand von Ausführungsbeispielen näher erläutert. Die Figuren zeigen:
- Figur 1: ein schematisches Flussdiagramm eines erfindungsgemäßen Verfahrens,
- Figur 2: ein schematisches Flussdiagramm des Bestimmens einer Referenzfläche,
- Figur 3a: eine schematische Ansicht einer Situsöffnung in einem Situs,
- Figur 3b: eine weitere schematische Ansicht einer Situsöffnung in einem Situs und
- Figur 4: eine schematische Ansicht eines erfindungsgemäßen Mikroskopiesystems.

Nachfolgend bezeichnen gleiche Bezugszeichenelemente mit gleichen oder ähnlichen technischen Merkmalen.

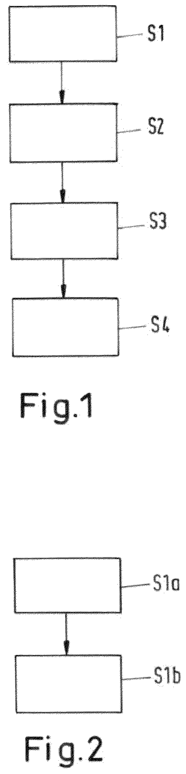

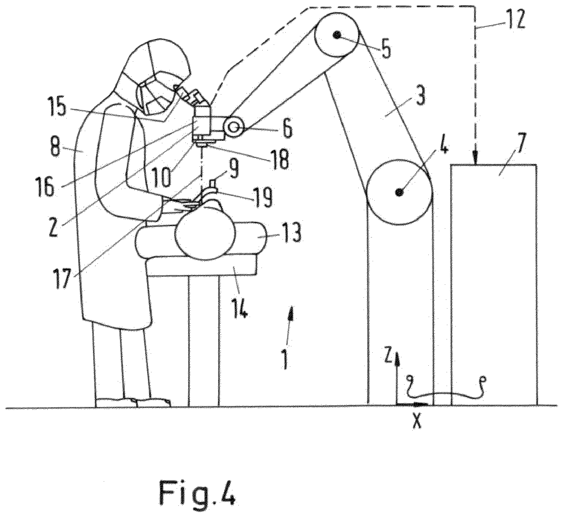

Figur 1 zeigt ein schematisches Flussdiagramm eines erfindungsgemäßen Verfahrens zum Betreiben eines Mikroskopiesystems 1 (siehe zum Beispiel Figur 4).

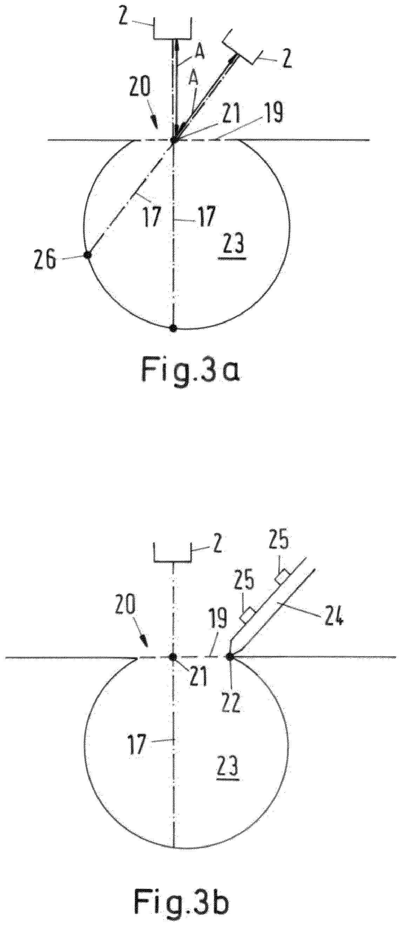

Hierbei erfolgt in einem ersten Schritt S1 das Bestimmen einer Referenzfläche 19 (siehe zum Beispiel Figur 3). Dies umfasst insbesondere das Bestimmen einer Lage, also zum Beispiel einer Position eines Stützpunkts der Referenzfläche 19 und eine Orientierung der Referenzfläche 19, im Referenzkoordinatensystem.

Das Bestimmen der Referenzfläche 19 erfolgt hierbei datenbasiert. Beispielsweise können zum Bestimmen präoperative Daten eingelesen werden, wobei die Referenzfläche 19 durch Auswertung der präoperativen Daten bestimmt wird. Hierzu kann es erforderlich sein, ein Koordinatensystem der präoperativen Daten in das vorhergehend erläuterte Referenzkoordinatensystem zu transformieren. Dies kann durch geeignete Registrierungsverfahren erfolgen.

Beispielsweise ist es möglich, durch Verfahren der Bildverarbeitung eine Situsöffnung 20 in den präoperative Daten zu detektieren, wobei diese Situsöffnung 20 beispielsweise eine geplante Situsöffnung 20 ist, die durch einen Operationsplaner, beispielsweise durch eine Nutzereingabe, festgelegt wird.

Dann kann die Referenzfläche 19 derart festgelegt werden, dass die von der detektierten Situsöffnung 20 umfasste Fläche die Referenzfläche 19 bildet oder von der Referenzfläche 19 umfasst wird.

Auch ist es möglich, topografischen Informationen des Situs, insbesondere intraoperativ, zu bestimmen, wobei dann die Daten, die die topografischen Informationen kodieren, ausgewertet werden können, um die Referenzfläche 19 festzulegen. Topografische Informationen können, wie vorhergehend bereits ausführlich erläutert, beispielsweise Bildinformationen, zum Beispiel in Form von 2-oder dreidimensionalen Abbildern des Situs, sein, wobei Bildinformationen durch eine oder mehrere Bilderfassungseinrichtung(en) erzeugt werden können. Beispielsweise ist es möglich, durch Verfahren der Bildverarbeitung eine Situsöffnung 20 in diesen intraoperativ erzeugten Bilddaten zu detektieren. Auch dann kann die Referenzfläche 19 derart festgelegt werden, dass die von der der diktierten Situsöffnung 20 umfasste Fläche die Referenzfläche 19 bildet oder von der Referenzfläche 19 umfasst wird.

Weiter kann das Bestimmen der Referenzfläche 19 auch das Bestimmen der Form der Referenzfläche 19 umfassen. Dieses Bestimmen der Form kann insbesondere auch durch Auswertung der erläuterten Daten erfolgen, beispielsweise durch die Anwendung geeigneter Verfahren zur Bildverarbeitung. Selbst verständlich ist es aber auch vorstellbar, dass die Form der Referenzfläche 19 eine vorbestimmte Form ist. Hierbei kann es möglich sein, dass alle Eigenschaften der Form vorbestimmt sind. Auch kann es möglich sein, dass nur bestimmte Eigenschaften der Form vorbestimmt sind, während weitere Eigenschaften der Form datenbasiert bestimmt werden. So kann beispielsweise festgelegt sein, dass die Form der Referenzfläche 19 eine Kreisform ist, wobei der Radius datenbasiert bestimmt wird.

Weiter wird die derart bestimmte Lage der Referenzfläche 19 in einem fokuslagenunabhängigen Referenzkoordinatensystem, insbesondere in dem in Fig. 4 dargestellten Referenzkoordinatensystem, in abrufbarer Weise abgespeichert. Die Lage der Referenzfläche kann hierbei auch in einem objektebenenunabhängigen und/oder schärfeebenenunabhängigen Referenzkoordinatensystem festgelegt und in abrufbarer Weise gespeichert sein.

Die Referenzfläche 19 muss also nur einmalig bestimmt werden, insbesondere wenn eine Lage eines Situs des Patienten 13 (siehe Fig. 4) sich nicht ändert. Dann können neue Drehpunkte, insbesondere nach Änderung der Lage der optischen Achse 17, jeweils als Schnittpunkt zwischen der gespeicherten Referenzfläche 19 und dieser optischen Achse 17 in ihrer aktuellen Lage, also aktuellen Position und aktuellen Orientierung, bestimmt werden.

Wurde die Lage der Referenzfläche 19 also bereits bestimmt so können alternativ zu der erläuterten Bestimmung im ersten Schritt S1 auch die gespeicherten Informationen über die Lage der Referenzfläche 19 aus einer Speichereinrichtung abgerufen werden.

Weiter erfolgt in einem zweiten Schritt S2 das Bestimmen einer Lage einer optischen Achse 17 eines Mikroskops 2. Dies umfasst insbesondere das Bestimmen der Orientierung der optischen Achse 17 sowie der Position eines Stützpunkts der optischen Achse 17, beispielsweise ein Schnittpunkt der optischen Achse 17 mit einer Linse des Mikroskops 2, in einem Referenzkoordinatensystem. Das Referenzkoordinatensystem wird mit Bezug auf Figur 4 nachfolgend noch näher erläutert. Die Orientierung der optischen Achse 17 kann insbesondere in Abhängigkeit von Ausgangssignalen von Lage-/Winkelsensoren des Mikroskopiesystems 1, insbesondere eines Stativs 3 des Mikroskopiesystems 1, bestimmt werden.

Weiter erfolgt in einem dritten Schritt S3 das Bestimmen eines Drehpunkts 21 für das Mikroskop 2 als Schnittpunkt zwischen der optischen Achse 17 und der Referenzfläche 19. Dies umfasst insbesondere das Bestimmen der Koordinaten dieses Drehpunkts 21 im Referenzkoordinatensystem.

In einem vierten Schritt S4 erfolgt das Betreiben von Antriebseinrichtungen und/oder Bremseinrichtungen eines Stativs 3 (siehe zum Beispiel Figur 4) des Mikroskopiesystems 1 derart, dass sich das Mikroskop 2 des Mikroskopiesystems 1 mit Abstand A (siehe zum Beispiel Figur 3) um den Drehpunkt 21 herumbewegt. Der Abstand A kann hierbei ein konstanter Abstand sein. Dies ist jedoch nicht zwingend. Hierzu können beispielsweise Bewegungen von beweglichen Teilen des Stativs 3 um Drehachse 4, 5, 6 gesperrt werden, wenn sich durch diese Bewegungen der Abstand A verändern würde. Weiter ist es möglich, dass nur solche Bewegungen freigegeben oder durch die Antriebseinrichtungen erzeugt werden, durch die das Mikroskop 2 mit konstantem Abstand A um den Drehpunkt 21 herumbewegt wird.

Dieses Betreiben kann von einer Steuereinrichtung 7 gesteuert/geregelt werden. Die Steuereinrichtung 7 kann auch das Bestimmen der Lage der optischen Achse 17, dass Bestimmen der Referenzfläche 19 und das Bestimmen des Schnittpunkts durchführen.

Figur 2 zeigt ein schematisches Flussdiagramm des Bestimmens der Referenzfläche 19. Hierbei erfolgt in einem ersten Teilschritt S1a ein Festlegen eines Referenzflächenpunkts 22 (siehe zum Beispiel Figur 3b). Dieser Referenzflächenpunkt 22 kann hierbei von dem zu bestimmenden Drehpunkt 21 verschieden sein. Verschiedene Möglichkeiten zur Festlegung eines Referenzflächenpunkts 22 wurden vorhergehend erläutert. Beispielsweise kann der Referenzflächenpunkt 22 als ein von einem Nutzer eingestellter Fokuspunkt des Mikroskops 2 bestimmt werden, wobei der Fokuspunkt hier nach wieder verändert werden kann. Ebenfalls ist es möglich, dass der Referenzflächenpunkt 22 in Abhängigkeit einer Lage mindestens eines Markers bestimmt wird, wobei diese Lage beispielsweise bildbasiert bestimmt werden kann. Weiter ist es möglich, dass der Referenzflächenpunkt 22 in Abhängigkeit einer Blickrichtungserkennung festgelegt wird. Informationen über den Referenzpunkt können in abrufbarer Form gespeichert werden und ermöglichen eine einfache erneute Bestimmung der Referenzfläche 19 und des Drehpunkts 21.

In einem weiteren Teilschritt 1b wird die Referenzfläche 19 als Ebene festgelegt oder als eine Fläche in der Ebene bestimmt, die senkrecht zur optischen Achse 17 des Mikroskops 2 orientiert ist und in der der Referenzflächenpunkt 22 angeordnet ist.

Es ist möglich, dass im ersten Teilschritt 1a mehrere, insbesondere mindestens drei, Referenzflächenpunkte 22 festgelegt werden, wobei dann die Lage und/oder die von der Referenzfläche 19 derart bestimmt wird, dass die mehreren Referenzflächenpunkte 22 in der Referenzfläche 19 liegen oder nicht mehr als ein vorbestimmtes Maß von der Referenzfläche 19 oder einer Ebene, die die Referenzfläche 19 enthält, beabstandet sind.

Figur 3a zeigt schematische Ansicht einer Situsöffnung 20 in einem Situs. Weiter dargestellt ist ein Mikroskop 2 sowie eine optische Achse 17 des Mikroskops 2. Hierbei ist das Mikroskop 2 in verschiedenen Orientierungen dargestellt. Weiter ersichtlich ist, dass ein Drehpunkt 21 in einer Referenzfläche 19 angeordnet ist, wobei die Lage und die Form der Referenzfläche 19 derart festgelegt ist, dass die von der Situsöffnung 20 umfasste Fläche die Referenzfläche 19 bildet.

Die Situsöffnung 20 kann beispielsweise eine Schädelöffnung sein, die bei einer neurochirurgischen Operation in den Schädel eines Patienten eingebracht wird, um Zugang zu darunterliegenden Strukturen zu erhalten. Regelmäßig wird hierbei eine Kavität 23 im Hirn geschaffen, deren Durchmesser größer als der Durchmesser der Schädelöffnung ist. Durch das Festlegen des Drehpunkts 21 als Punkt der erläuterten Referenzfläche 19 ergibt sich in vorteilhafter Weise, dass eine Bodenfläche der Kavität mit verschiedenen Blickrichtungen durch das Mikroskop 2 von einem Nutzer, beispielsweise dem Neurochirurgen, betrachtet werden kann, ohne dass die Sicht verdeckt wird, beispielsweise durch die Schädelstruktur, die die Schädelöffnung umfasst, wobei zusätzlich ermöglicht wird, dies mit einer Bewegung mit Anteilen in nur wenig Freiheitsgraden zu realisieren. Weiter ist es möglich, einen Fokuspunkt 24 des Mikroskops 2 unabhängig von dem Drehpunkt 21 einzustellen. Insbesondere kann also eine Position des Fokuspunkts 24 verändert werden, ohne dass die Position des Drehpunkts 21 verändert wird.

Figur 3b zeigt eine weitere schematische Ansicht einer Situsöffnung 20 in einem Situs. Ebenfalls dargestellt ist ein Mikroskop 2 sowie eine optische Achse 17 des Mikroskops 2. Weiter ersichtlich ist, dass ein Drehpunkt 21 in einer Referenzfläche 19 angeordnet ist, wobei die Lage und die Form der Referenzfläche 19 derart festgelegt ist, dass die von der Situsöffnung 20 umfasste Fläche die Referenzfläche 19 bildet.

Weiter dargestellt ein Lagemarkierungsinstrument 24, welches zum Beispiel von einem Nutzer positioniert werden kann. Durch eine Spitze des Lagemarkierungsinstruments 24 ist eine zum Beispiel durch eine Lageerfassungseinrichtung (nicht dargestellt) erfassbare Lage des Lagemarkierungsinstruments 24 festgelegt. Schematisch dargestellt sind optisch erfassbare Markerelemente 25, die durch die Lageerfassungseinrichtung erfasst werden können, wobei dann in Abhängigkeit dieser abgebildeten Markerelemente 25 die durch die Spitze festgelegte Lage bestimmt werden kann. Die Markerelemente 25 können Teil eines Targets 9 (siehe Fig. 4) sein. Weiter dargestellt ist, dass durch das Lagemarkierungsinstrument 24 der Referenzflächenpunkt 22 festgelegt ist. Durch diesen wird die Referenzfläche 19 festgelegt, die gleichzeitig orthogonal zur optischen Achse 17 des Mikroskops 2 orientiert ist.

Figur 4 zeigt eine schematische Ansicht eines erfindungsgemäßen Mikroskopiesystems 1. Das Mikroskopiesystem 1 umfasst ein Mikroskop 2, welches an einem Stativ 3 zur Halterung des Mikroskops 2 angeordnet ist, insbesondere an einem freien Ende des Stativs 3. Das Stativ 3 ermöglicht eine Bewegung des Mikroskops 2 zur Veränderung der Lage, also der Position und/oder Orientierung des Mikroskops 2. Dargestellt ist ein Referenzkoordinatensystem mit einer Vertikalachse z und einer Längsachse x. Die Vertikalachse z ist hierbei parallel zur Richtung einer Gravitationskraft und entgegengesetzt zu dieser orientiert. Die Längsachse x ist senkrecht zur Vertikalachse z orientiert. Eine nicht dargestellte Querachse des Referenzkoordinatensystem ist hierbei senkrecht zur Längs- und Vertikalachse x, z orientiert, wobei die Achsen x, z ein kartesisches Koordinatensystem bilden.

Das dargestellte Stativ 3 stellt eine exemplarische kinematische Struktur zur Halterung und Bewegung des Mikroskops 2 dar. Dem Fachmann ist selbstverständlich bekannt, dass auch andere kinematische Strukturen verwendet werden können.

Das Stativ 3 umfasst nicht dargestellte Antriebseinrichtungen zur Bewegung des Mikroskops 2. Hierbei können die Antriebseinrichtungen beispielsweise eine Drehbewegung von beweglichen Teilen des Stativs 3 um Drehachsen 4, 5, 6 und eine Drehachse parallel zur Vertikalachse z ermöglichen. Weiter dargestellt ist eine Steuereinrichtung 7, die zur Steuerung der nicht dargestellten Antriebseinrichtungen dient und die beispielsweise einen Mikrocontroller umfassen kann. Die Steuereinrichtung 7 kann hierbei eine Steuer- und Auswerteeinrichtung bilden.

Weiter kann die Steuereinrichtung 7 auch nicht dargestellte Bremseinrichtungen des Stativs 3 steuern, wobei diese die Drehbewegung der beweglichen Teile bremsen oder verhindern können.

Mittels der Steuereinrichtung 7 können die Antriebseinrichtungen insbesondere derart angesteuert werden, dass das Mikroskop 2 eine gewünschte Bewegung ausführt, insbesondere im Referenzkoordinatensystem. Beispielsweise ist es möglich, das Mikroskop 2 in eine gewünschte Raumposition mit einer gewünschten Orientierung zu positionieren. Weiter kann die Steuereinrichtung 7 auch zur Einstellung von Betriebs- und/oder Bewegungsparametern des Mikroskops 2 dienen, beispielsweise von einem Fokuswert des Mikroskops 2. Hierzu kann die Steuereinrichtung 7 signal- und/oder datentechnisch mit dem Mikroskop 2 und/oder mit den Antriebseinrichtungen verbunden sein.

Das Mikroskopiesystem 1 umfasst weiter eine Lageerfassungseinrichtung zur Erfassung einer Lage eines Instruments 19, welches von einem Benutzer 8 gehalten und bewegt werden kann. Das Instrument 19 kann insbesondere ein Lagemarkierungsinstrument 24 sein, welches z.B. in Fig. 3b dargestellt ist. Der Benutzer 8 kann beispielsweise ein Chirurg sein. Die Lageerfassungseinrichtung umfasst mindestens ein Target 9 mit mindestens einem Markerelement 25 (siehe z.B. Fig. 4) und mindestens eine Bilderfassungseinrichtung 10 zur Erfassung des Targets. Mittels der Lageerfassungseinrichtung kann eine Lage des Targets 9 relativ zur Bilderfassungseinrichtung bestimmt werden, insbesondere in einem Koordinatensystem der Lageerfassungseinrichtung. Hierbei umfasst das Target 9 mindestens ein passives Markerelement 25, vorzugsweise drei passive Markerelemente 25.

In Fig. 4 ist dargestellt, dass das Target 9 an dem Instrument 19 befestigt ist. Das Instrument 19 kann z.B. als Sauger ausgebildet sein. Das Instrument 19 wird hierbei derart vom Benutzer 8 gehalten, dass das Target 9 in einem Erfassungsbereich der Bilderfassungseinrichtung 10 angeordnet ist.

Die Lage des Instruments 19 kann mit der Lagerfassungseinrichtung erfasst werden, indem die Lage des Targets 9, insbesondere bildbasiert, bestimmt wird, wobei dann aufgrund der ortsfesten Anordnung des Targets 9 an dem Instrument 19 auch die Lage des Instruments 19 bestimmt werden kann. Eine Relativlage zwischen Target 9 und Instrument 19 kann hierbei vorbekannt sein und z.B. durch eine Registrierung bestimmt werden.

Weiter dargestellt ist eine Bilderfassungseinrichtung 10 des Mikroskopiesystems 1, beispielsweise eine CCD-Kamera. Diese Bilderfassungseinrichtung 10 ist in einem Mikroskopkörper 16 des Mikroskops 2 angeordnet. Insbesondere ist die Bilderfassungseinrichtung in einem Gehäuse des Mikroskopkörpers 16 angeordnet. Weiter insbesondere ist die Bilderfassungseinrichtung 10 mechanisch starr an einem Teil des Mikroskops 2 und somit ortsfest relativ zu diesem Teil angeordnet.

Weiter dargestellt ist eine signal- und/oder datentechnische Verbindung 12 zwischen der Bilderfassungseinrichtung 10 und der Steuereinrichtung 7. Mittels der Steuereinrichtung 7 oder mittels einer nicht dargestellten Auswerteeinrichtung, die beispielsweise Teil der Lageerfassungseinrichtung sein kann, kann eine Relativlage zwischen Target 9 und Bilderfassungseinrichtung 10 in einem dreidimensionalen Koordinatensystem der Lageerfassungseinrichtung bestimmt werden. So kann z.B. die Lage des Targets 9 in einem zweidimensionalen Bildkoordinatensystem der Bilderfassungseinrichtung 10 bestimmt werden und in Abhängigkeit dieser Lage dann eine Lage in dem Koordinatensystem der Lageerfassungseinrichtung. Hierbei kann sowohl eine Position als auch eine Orientierung in dem dreidimensionalen Koordinatensystem der Lageerfassungseinrichtung bestimmt werden. Durch die Befestigung des Targets 9 an dem Instrument 19 kann somit auch eine Lage des Instruments 19 im Koordinatensystem der Lageerfassungseinrichtung und somit auch im Referenzkoordinatensystem bestimmt werden. Insbesondere kann mittels der Lageerfassungseinrichtung auch eine Lageänderung des Targets 9 und somit auch eine Lageänderung des Instruments 19 erfasst werden.

Zeitlich vor dem Betrieb des Mikroskopiesystems 1 kann das Koordinatensystem der Lageerfassungseinrichtung mit dem dargestellten Referenzkoordinatensystem registriert werden. Mit anderen Worten kann eine Transformationsvorschrift zur Transformation der Lage im Koordinatensystem der Lageerfassungseinrichtung in das Referenzkoordinatensystem bestimmt werden.

In Fig. 4 ist dargestellt, dass die Bilderfassungseinrichtung 10 in dem Mikroskopkörper 16 angeordnet ist. Selbstverständlich ist es auch möglich, diese außerhalb des Mikroskopkörpers 16 an diesem zu befestigen. Auch ist es möglich, die Bilderfassungseinrichtung nicht am Mikroskopiesystem 1 zu befestigen, sondern auf einem Stativ der Lageerfassungseinrichtung, das vom Stativ 3 des Mikroskopiesystems 1 verschieden ist.

Die Erfassung der Lage des Targets 9 kann durch Auswertung genau eines zweidimensionalen Abbilds der Bilderfassungseinrichtung 10 erfolgen.

Weiter dargestellt ist ein Patient 13, der auf einem Operationstisch 14 liegt. Weiter dargestellt ist, dass das Mikroskop 2 ein Okular 15 umfasst, in welches der Benutzer 8 hineinschaut, um durch das Mikroskop 2 einen Teilbereich des Patienten 13 zu betrachten, insbesondere in vergrößernder Art und Weise.

Dargestellt ist auch eine optische Achse 17 des Mikroskops 2. In einer Strahlrichtung entlang dieser optischen Achse vom Mikroskop 2 zum Patienten 13 ist die Bilderfassungseinrichtung 10 vor einer Glasscheibe 18 des Mikroskops 2 angeordnet, die das Innere des Gehäuses des Mikroskopkörpers 16 gegenüber der äußeren Umgebung abschließt. Somit ist die Glasscheibe 18 zwischen der Bilderfassungseinrichtung 10 und dem zu betrachtenden Patienten 13 angeordnet.

Ein Erfassungsbereich der Bilderfassungseinrichtung 10 zur Lageerfassung überlappt hierbei zumindest teilweise mit einem Erfassungsbereich des Mikroskops zur vergrößerten Darstellung des Patienten oder Körperbereichen des Patienten 13.

Es ist möglich, dass der Benutzer 8 durch die Bewegung des Instruments 19, beispielsweise mit seinen Händen, das Target 9 bewegt und somit dessen Lage ändert. Die Lageänderung kann hierbei durch die Lageerfassungseinrichtung erfasst werden, wobei dann eine derart eingestellte Lage als Lage eines Referenzflächenpunkts 22 (siehe Fig. 3b) bestimmt werden kann.

Nicht dargestellt sind Winkelsensoren zur Erfassung einer Relativlage zwischen den beweglichen Teilen des Stativs 7, wobei die Steuereinrichtung 7 in Abhängigkeit von Ausgangssignalen der Winkelsensoren eine räumliche Lage des Mikroskops 2, insbesondere auch der optischen Achse 17 bestimmen kann.

Mittels der Steuereinrichtung 7 ist das Mikroskopiesystems derart betreibbar, dass sich das Mikroskop 2 mit einem konstanten Abstand A (siehe Fig. 3a) um einen Drehpunkt 21 herumbewegt, wobei der Drehpunkt 21 ein Schnittpunkt der optischen Achse 17 des Mikroskops 2 und einer Referenzfläche 19 ist. Weiter ist die Referenzfläche 19 datenbasiert bestimmbar, insbesondere mittels der Steuereinrichtung 7. Dies wurde vorhergehend bereits erläutert.

### Bezugszeichenliste

- 1: Mikroskopiesystem
- 2: Mikroskop
- 3: Stativ
- 4: Drehachse
- 5: Drehachse
- 6: Drehachse
- 7: Steuereinrichtung
- 8: Benutzer
- 9: Target
- 10: Bilderfassungseinrichtung
- 12: signal- und/oder datentechnische Verbindung
- 13: Patient
- 14: Operationstisch
- 15: Okular
- 16: Mikroskopkörper
- 17: optische Achse
- 18: Glasscheibe
- 19: Instrument
- 20: Situsöffnung
- 21: Drehpunkt
- 22: Referenzflächenpunkt
- 23: Kavität
- 24: Lagemarkierungsinstrument
- 25: Markerelement
- 26: Fokuspunkt
- A: Abstand
- S1: erster Schritt
- S2: zweiter Schritt
- S2a: erster Teilschritt
- S2b: zweiter Teilschritt
- S3: dritter Schritt
- S4: vierter Schritt

## Patentansprüche

1. Verfahren zum Betreiben eines Mikroskopiesystems (1), wobei ein Drehpunkt (21) festgelegt wird, wobei das Mikroskopiesystems (1) derart betrieben wird, dass sich ein Mikroskop (2) des Mikroskopiesystems (1) um den Drehpunkt (21) herum bewegt, wobei als Drehpunkt (21) ein Schnittpunkt einer optischen Achse (17) des Mikroskops (2) und einer Referenzfläche (19) bestimmt wird, wobei die Lage der Referenzfläche (19) in einem fokuslagenunabhängigen Referenzkoordinatensystem festgelegt ist und der Drehpunkt (21) als der Schnittpunkt der optischen Achse (17) mit der derart festgelegten Referenzfläche im Referenzkoordinatensystem bestimmt wird, wobei die Referenzfläche (19) bestimmt wird, **dadurch gekennzeichnet, dass**
Informationen über eine Lage der Referenzfläche (19) in abrufbarer Form gespeichert werden, wobei zur Bestimmung des Drehpunkts (21) nach einer Änderung der Lage der optischen Achse (17) die abgespeicherten Informationen über die Referenzfläche (19) abgerufen werden und der Drehpunkt (21) als Schnittpunkt der optischen Achse (17) in ihrer aktuellen Lage mit der abgespeicherten Referenzfläche (19) bestimmt wird.

2. Verfahren nach Anspruch 1, **dadurch gekennzeichnet, dass** die Lage der Referenzfläche (19) in einem objektebenenunabhängigen Referenzkoordinatensystem festgelegt ist.

3. Verfahren nach Anspruch 1 oder 2, **dadurch gekennzeichnet, dass** die Lage und/oder die Form der Referenzfläche (19) derart festgelegt wird, dass die von einer Situsöffnung (20) umfassten Fläche die Referenzfläche (19) bildet oder von der Referenzfläche (19) zumindest teilweise umfasst wird.

4. Verfahren nach einem der vorangegangenen Ansprüche, **dadurch gekennzeichnet, dass** die Situsöffnung (20) automatisch oder manuell festgelegt wird.

5. Verfahren nach einem der vorangegangenen Ansprüche, **dadurch gekennzeichnet, dass** die Referenzfläche (19) eine begrenzte Fläche ist und/oder eine gekrümmte Fläche ist und/oder die Lage und/oder die Form der Referenzfläche (19) in Abhängigkeit von präoperativ erzeugten Daten bestimmt wird.

6. Verfahren nach einem der vorangegangenen Ansprüche, **dadurch gekennzeichnet, dass** topographische Informationen eines Situs bestimmt werden, wobei die Lage und/oder Form der Referenzfläche (19) in Abhängigkeit dieser topographischen Informationen bestimmt wird.

7. Verfahren nach einem der vorangegangenen Ansprüche, **dadurch gekennzeichnet, dass** die Referenzfläche (19) bestimmt wird, indem mindestens ein Referenzflächenpunkt (22) festgelegt wird, wobei die Referenzfläche (19) als Ebene festgelegt wird oder in der Ebene angeordnet ist, die senkrecht zur optischen Achse (17) des Mikroskops (2) orientiert ist und in der der Referenzflächenpunkt (22) angeordnet ist.

8. Verfahren nach Anspruch 7, **dadurch gekennzeichnet, dass** der mindestens eine Referenzflächenpunkt (22) als von einem Nutzer eingestellter Fokuspunkt bestimmt wird oder dass der mindestens eine Referenzflächenpunkt (22) durch eine Positionierung eines Lagemarkierungsinstruments (24) festgelegt wird oder dass der Referenzflächenpunkt (22) in Abhängigkeit einer Position und/oder Orientierung mindestens eines Markers bestimmt wird oder dass der mindestens eine Referenzflächenpunkt (22) in Abhängigkeit einer Blickrichtungserkennung festgelegt wird.

9. Verfahren nach Anspruch 7 oder 8, **dadurch gekennzeichnet, dass** die Lage eines Referenzflächenpunkts (22) festgelegt wird, indem die Lage eines bereits festgelegten Referenzflächenpunkts (22) geändert wird.

10. Verfahren nach einem der Ansprüche 7 bis 9, **dadurch gekennzeichnet, dass** mindestens drei Referenzflächenpunkte (22) bestimmt werden, wobei die Lage und/oder die Form der Referenzfläche (19) derart bestimmt wird, dass die mindestens drei Referenzflächenpunkte (22) in der Referenzfläche (19) liegen oder ein Abstand der Referenzflächenpunkte von der Referenzfläche oder einer Ebene, die die Referenzfläche enthält, minimal ist.

11. Verfahren nach Anspruch 5, **dadurch gekennzeichnet, dass** als Drehpunkt (21) der Fokuspunkt festgelegt wird, falls die optische Achse (17) die begrenzte Referenzfläche (19) nicht schneidet.

12. Verfahren nach einem der vorangegangenen Ansprüche, **dadurch gekennzeichnet, dass** eine Fokuslage in Abhängigkeit der Änderung des Abstands des Mikroskops (2) vom Drehpunkt (21) geändert wird.

13. Mikroskopiesystem, umfassend:
• ein Mikroskop (2),
• ein Stativ (3) zur Halterung des Mikroskops (2),
• mindestens eine Steuer- und Auswerteeinrichtung (7) zur Steuerung des Mikroskopiesystems (1), wobei das Mikroskopiesystem (1) derart betreibbar ist, dass sich das Mikroskop (2) um einen Drehpunkt (21) herumbewegt, wobei eine Referenzfläche (19) bestimmbar ist, wobei als Drehpunkt (21) ein Schnittpunkt einer optischen Achse (17) des Mikroskops (2) und einer Referenzfläche (19) bestimmbar ist, wobei die Lage der Referenzfläche (19) in einem fokuslagenunabhängigen Referenzkoordinatensystem festgelegt ist und der Drehpunkt (21) als der Schnittpunkt der optischen Achse (17) mit der derart festgelegten Referenzfläche im Referenzkoordinatensystem bestimmbar ist, wobei die Referenzfläche (19) bestimmt wird,
**dadurch gekennzeichnet, dass**
Informationen über eine Lage der Referenzfläche (19) in abrufbarer Form gespeichert werden, wobei zur Bestimmung des Drehpunkts (21) nach Änderung einer Lage der optischen Achse (17) die abgespeicherten Informationen über die Referenzfläche (19) abgerufen werden und der Drehpunkt (21) als Schnittpunkt der optischen Achse (17) in ihrer aktuellen Lage mit der abgespeicherten Referenzfläche (19) bestimmt wird.

14. Mikroskopiesystem nach Anspruch 13, **dadurch gekennzeichnet, dass** die Lage der Referenzfläche (19) in einem objektebenenunabhängigen Referenzkoordinatensystem festgelegt ist.

15. Mikroskopiesystem nach Anspruch 13 oder 14, **dadurch gekennzeichnet, dass** das Mikroskopiesystem (1) eine Einrichtung zur Erzeugung topographischer Informationen und/oder mindestens eine Lageerfassungseinrichtung zur Erfassung einer Lage eines Lagemarkierungsinstruments (24) und/oder eines Markers umfasst.

## Claims

1. Method for operating a microscopy system (1), wherein a pivot point (21) is defined, wherein the microscopy system (1) is operated in such a way that a microscope (2) of the microscopy system (1) moves around the pivot point (21), wherein an intersection of an optical axis (17) of the microscope (2) and a reference surface (19) is determined as the pivot point (21), wherein the pose of the reference surface (19) is defined in a focal position-independent reference coordinate system and the pivot point (21) is determined as the intersection of the optical axis (17) with the thus defined reference surface in the reference coordinate system, wherein the reference surface (19) is determined,
**characterized in that**
information about a pose of the reference surface (19) is stored in a retrievable form, wherein the stored information about the reference surface (19) is retrieved in order to determine the pivot point (21) after a change in the pose of the optical axis (17), and the pivot point (21) is determined as the intersection of the optical axis (17) in its current pose with the stored reference surface (19).

2. Method according to Claim 1, **characterized in that** the pose of the reference surface (19) is defined in an object plane-independent reference coordinate system.

3. Method according to Claim 1 or 2, **characterized in that** the pose and/or the shape of the reference surface (19) is defined in such a way that the area encompassed by a situs opening (20) forms the reference surface (19) or is at least partially encompassed by the reference surface (19).

4. Method according to any one of the preceding claims, **characterized in that** the situs opening (20) is defined automatically or manually.

5. Method according to any one of the preceding claims, **characterized in that** the reference surface (19) is a bounded surface and/or a curved surface, and/or the pose and/or the shape of the reference surface (19) is determined on the basis of preoperatively generated data.

6. Method according to any one of the preceding claims, **characterized in that** topographical information of a situs is determined, the pose and/or shape of the reference surface (19) being determined on the basis of this topographical information.

7. Method according to any one of the preceding claims, **characterized in that** the reference surface (19) is determined by defining at least one reference surface point (22), with the reference surface (19) being defined as or being arranged in the plane which is oriented perpendicular to the optical axis (17) of the microscope (2) and in which the reference surface point (22) is arranged.

8. Method according to Claim 7, **characterized in that** the at least one reference surface point (22) is determined as a focal point set by a user or **in that** the at least one reference surface point (22) is defined by a positioning of a pose marking instrument (24) or **in that** the reference surface point (22) is determined on the basis of a position and/or orientation of at least one marker or **in that** the at least one reference surface point (22) is defined on the basis of a viewing direction detection.

9. Method according to Claim 7 or 8, **characterized in that** the pose of a reference surface point (22) is defined by changing the pose of an already defined reference surface point (22).

10. Method according to any one of Claims 7 to 9, **characterized in that** at least three reference surface points (22) are determined, the pose and/or the shape of the reference surface (19) being determined in such a way that the at least three reference surface points (22) are located on the reference surface (19) or a distance of the reference surface points from the reference surface or a plane containing the reference surface is minimal.

11. Method according to Claim 5, **characterized in that** the focal point is defined as the pivot point (21) if the optical axis (17) does not intersect the bounded reference surface (19).

12. Method according to any one of the preceding claims, **characterized in that** a focal position is changed on the basis of the change in the distance of the microscope (2) from the pivot point (21).

13. Microscopy system comprising:
• a microscope (2),
• a stand (3) for holding the microscope (2),
• at least one control and evaluation device (7) for controlling the microscopy system (1), wherein the microscopy system (1) is operable in such a way that the microscope (2) moves around a pivot point (21), wherein a reference surface (19) is determinable, wherein an intersection of an optical axis (17) of the microscope (2) and a reference surface (19) is determinable as the pivot point (21), wherein the pose of the reference surface (19) is defined in a focal position-independent reference coordinate system and the pivot point (21) is determinable as the intersection of the optical axis (17) with the thus defined reference surface in the reference coordinate system, wherein the reference surface (19) is determined,
**characterized in that**
information about a pose of the reference surface (19) is stored in a retrievable form, wherein the stored information about the reference surface (19) is retrieved in order to determine the pivot point (21) after the change in a pose of the optical axis (17), and the pivot point (21) is determined as the intersection of the optical axis (17) in its current pose with the stored reference surface (19).

14. Microscopy system according to Claim 13, **characterized in that** the pose of the reference surface (19) is defined in an object plane-independent reference coordinate system.

15. Microscopy system according to Claim 13 or 14, **characterized in that** the microscopy system (1) comprises a device for generating topographical information and/or at least one pose detection device for detecting a pose of a pose marking instrument (24) and/or a marker.

## Revendications

1. Procédé d'exploitation d'un système de microscopie (1), un point de rotation (21) étant défini, le système de microscopie (1) étant exploité de telle sorte qu'un microscope (2) du système de microscopie (1) se déplace autour du point de rotation (21), un point d'intersection d'un axe optique (17) du microscope (2) et d'une surface de référence (19) étant déterminé en tant que point de rotation (21), la position de la surface de référence (19) étant définie dans un système de coordonnées de référence indépendant de la position focale et le point de rotation (21) étant déterminé en tant que point d'intersection de l'axe optique (17) avec la surface de référence ainsi définie dans le système de coordonnées de référence, la surface de référence (19) étant déterminée, **caractérisé en ce que**
des informations sur une position de la surface de référence (19) sont mémorisées sous forme interrogeable, les informations mémorisées sur la surface de référence (19) étant interrogées pour déterminer le point de rotation (21) après une modification de la position de l'axe optique (17) et le point de rotation (21) étant déterminé en tant que point d'intersection de l'axe optique (17) dans sa position actuelle avec la surface de référence (19) mémorisée.

2. Procédé selon la revendication 1, **caractérisé en ce que** la position de la surface de référence (19) est définie dans un système de coordonnées de référence indépendant du plan de l'objet.

3. Procédé selon la revendication 1 ou 2, **caractérisé en ce que** la position et/ou la forme de la surface de référence (19) est définie de telle sorte que la surface entourée par une ouverture de site (20) forme la surface de référence (19) ou est au moins partiellement entourée par la surface de référence (19).

4. Procédé selon l'une quelconque des revendications précédentes, **caractérisé en ce que** l'ouverture de site (20) est définie automatiquement ou manuellement.

5. Procédé selon l'une quelconque des revendications précédentes, **caractérisé en ce que** la surface de référence (19) est une surface limitée et/ou est une surface incurvée et/ou la position et/ou la forme de la surface de référence (19) est déterminée en fonction de données générées en préopératoire.

6. Procédé selon l'une quelconque des revendications précédentes, **caractérisé en ce que** des informations topographiques d'un site sont déterminées, la position et/ou la forme de la surface de référence (19) étant déterminée en fonction de ces informations topographiques.

7. Procédé selon l'une quelconque des revendications précédentes, **caractérisé en ce que** la surface de référence (19) est déterminée en définissant au moins un point de surface de référence (22), la surface de référence (19) étant définie en tant que plan ou étant agencée dans le plan orienté perpendiculairement à l'axe optique (17) du microscope (2) et dans lequel est agencé le point de surface de référence (22).

8. Procédé selon la revendication 7, **caractérisé en ce que** l'au moins un point de surface de référence (22) est déterminé en tant que point focal réglé par un utilisateur ou **en ce que** l'au moins un point de surface de référence (22) est défini par un positionnement d'un instrument de marquage de position (24) ou **en ce que** le point de surface de référence (22) est déterminé en fonction d'une position et/ou d'une orientation d'au moins un marqueur ou **en ce que** l'au moins un point de surface de référence (22) est défini en fonction d'une détection de la direction du regard.

9. Procédé selon la revendication 7 ou 8, **caractérisé en ce que** la position d'un point de surface de référence (22) est défini en modifiant la position d'un point de surface de référence (22) déjà défini.

10. Procédé selon l'une quelconque des revendications 7 à 9, **caractérisé en ce qu'**au moins trois points de surface de référence (22) sont déterminés, la position et/ou la forme de la surface de référence (19) étant déterminée de telle sorte que les au moins trois points de surface de référence (22) soient situés dans la surface de référence (19) ou qu'une distance des points de surface de référence par rapport à la surface de référence ou à un plan contenant la surface de référence soit minimale.

11. Procédé selon la revendication 5, **caractérisé en ce que** le point focal est défini en tant que point de rotation (21) si l'axe optique (17) ne coupe pas la surface de référence limitée (19).

12. Procédé selon l'une quelconque des revendications précédentes, **caractérisé en ce qu'**une position focale est modifiée en fonction de la variation de la distance du microscope (2) par rapport au point de rotation (21).

13. Système de microscopie, comprenant :
- un microscope (2),
- un statif (3) pour le maintien du microscope (2),
- au moins un dispositif de commande et d'évaluation (7) pour la commande du système de microscopie (1), le système de microscopie (1) pouvant être exploité de telle sorte que le microscope (2) se déplace autour d'un point de rotation (21), une surface de référence (19) pouvant être déterminée, un point d'intersection d'un axe optique (17) du microscope (2) et d'une surface de référence (19) pouvant être déterminé en tant que point de rotation (21), la position de la surface de référence (19) étant définie dans un système de coordonnées de référence indépendant de la position focale et le point de rotation (21) pouvant être déterminé en tant que point d'intersection de l'axe optique (17) avec la surface de référence ainsi définie dans le système de coordonnées de référence, la surface de référence (19) étant déterminée,
**caractérisé en ce que**
des informations sur une position de la surface de référence (19) sont mémorisées sous forme interrogeable, les informations mémorisées sur la surface de référence (19) étant interrogées pour déterminer le point de rotation (21) après modification d'une position de l'axe optique (17) et le point de rotation (21) étant déterminé en tant que point d'intersection de l'axe optique (17) dans sa position actuelle avec la surface de référence (19) mémorisée.

14. Système de microscopie selon la revendication 13, **caractérisé en ce que** la position de la surface de référence (19) est définie dans un système de coordonnées de référence indépendant du plan de l'objet.

15. Système de microscopie selon la revendication 13 ou 14, **caractérisé en ce que** le système de microscopie (1) comprend un dispositif de génération d'informations topographiques et/ou au moins un dispositif de détection de position pour détecter une position d'un instrument de marquage de position (24) et/ou d'un marqueur.
